# EUROPEAN PATENT SPECIFICATION

(11) **EP 1 931 790 B1**
(45) Date of publication and mention of the grant of the patent: **25.01.2012**
(21) Application number: 06825300.4
(22) Date of filing: 02.10.2006
(51) Int. Cl.: C12N 15/82

(54) **TRANSGENIC PLANT SEED WITH INCREASED LYSINE**
TRANSGENE PFLANZENSAMEN MIT ERHÖHTEM LYSIN
GRAINE DE PLANTE TRANSGÉNIQUE À TENEUR AUGMENTÉE EN LYSINE

(30) Priority: 03.10.2005 US 723178 P
(43) Date of publication of application: 18.06.2008
(73) Proprietor: Monsanto Technology, LLC, St. Louis, MO 63167 (US)
(72) Inventor: MALVAR, Thomas, M., North Stonington, CT 06359 (US); HUANG, Shihshieh, Stonington, CT 06378 (US); LUETHY, Michael, H., Mystic, CT 06355 (US)
(74) Representative: von Kreisler Selting Werner
(86) International application number: PCT/US2006/038289
(87) International publication number: WO 2007/041419

(56) References cited:
- WO-A-93/19190
- WO-A-95/15392
- WO-A-2005/077117
- US-A1- 2003 056 242
- SHAUL O ET AL: "Increased lysine synthesis in tobacco plants that express high levels of bacterial dihydrodipicolinate synthase in their chloroplasts" PLANT JOURNAL, BLACKWELL SCIENTIFIC PUBLICATIONS, OXFORD, GB, vol. 2, no. 2, March 1992 (1992-03), pages 203-209, XP002261400 ISSN: 0960-7412
- FALCO S C ET AL: "TRANSGENIC CANOLA AND SOYBEAN SEEDS WITH INCREASED LYSINE" BIO/TECHNOLOGY, NATURE PUBLISHING CO. NEW YORK, US, vol. 13, no. 6, June 1995 (1995-06), pages 577-582, XP002035500 ISSN: 0733-222X

### SEQUENCE LISTING

<110> MALVAR, THOMAS M.
   HUANG, SHIHSHIEH
   LUETHY, MICHAEL H.
<120> TRANSGENIC PLANT SEED WITH INCREASED LYSINE
<130> MONS:094US
<140> UNKNOWN
   <141> 2006-09-22
<150> 60/723,178
   <151> 2005-10-03
<160> 11
<210> 1
   <211> 903
   <212> DNA
   <213> Corynebacterium glutamicum
<400> 1
   agtacaggtt taacagctaa gaccggagta gagcacttcg gcaccgttgg agtagcaatg
   60
   gttactccat tcacggaatc cggagacatc gatatcgctg ctggccgcga agtcgcggct
   120
   tatttggttg ataagggctt ggattctttg gttctcgcgg gcaccactgg tgaatcccca
   180
   acgacaaccg ccgctgaaaa actagaactg ctcaaggccg ttcgtgagga agttggggat
   240
   cgggcgaagc tcatcgccgg tgtcggaacc aacaacacgc ggacatctgt ggaacttgcg
   300
   gaagctgctg cttctgctgg cgcagacggc cttttagttg taactcctta ttactccaag
   360
   ccgagccaag agggattgct ggcgcacttc ggtgcaattg ctgcagcaac agaggttcca
   420
   atttgtctct atgacattcc tggtcggtca ggtattccaa ttgagtctga taccatgaga
   480
   cgcctgagtg aattacctac gattttggcg gtcaaggacg ccaagggtga cctcgttgca
   540
   gccacgtcat tgatcaaaga aacgggactt gcctggtatt caggcgatga cccactaaac
   600
   cttgtttggc ttgctttggg cggatcaggt ttcatttccg taattggaca tgcagccccc
   660
   acagcattac gtgagttgta cacaagcttc gaggaaggcg acctcgtccg tgcgcgggaa
   720
   atcaacgcca aactatcacc gctggtagct gcccaaggtc gcttgggtgg agtcagcttg
   780
   gcaaaagctg cttcgcgtct gcagggcatc aacgtaggag atcctcgact tccaattatg
   840
   gctccaaatg agcaggaact tgaggctctc cgagaagaca tgaaaaaagc tggagttcta
   900
   taa
   903
<210> 2
   <211> 3183
   <212> DNA
   <213> Zea mays
<400> 2
   atgggttctg ctgctactga gggcaatgac accttgctgg gcaatggagt tgttgggatt
   60
   cttgctgaga cttgtaatat gtgggaaaga agggcgccgt taactccttc ccattgtgcc
   120
   cgccttctgc taggaggagg caagaacgga cctcgagtaa accggattat tgtgcagcca
   180
   agcacaagga ggatccatca tgacgctcag tatgaggatg caggatgcga gatttcagaa
   240
   gacctgtcag aatgcggcct tatcataggc atcaaacaac ccaagctgca gatgattctt
   300
   tcagatagag cgtacgcttt cttttcacac acacacaaag cccaaaaaga gaatatgcca
   360
   ctgttagaca agatccttga agaaagggtg tccttgtttg attatgagct aattgttgga
   420
   gatgatggga aaagatcact agcatttggg aaatttgctg gtagagctgg actgatagat
   480
   ttcttacatg gtctcggaca gcgatatttg agccttggat actcgactcc atttctctct
   540
   ctgggacaat ctcatatgta tccttcgctc gctgcagcca aggctgcagt cattgtcgtt
   600
   gcagaagaga tagcaacatt tggacttcca tccggaattt gtccgatagt gtttgtgttc
   660
   actggagttg gaaacgtctc tcagggtgcg caggagatat tcaagttatt gccccatacc
   720
   tttgttgatg ctgagaagct tcccgaaatt tttcaggcca ggaatctgtc taagcaatct
   780
   cagtcgacca agagagtatt tcaactttat ggttgtgttg tgacctctag agacatggtt
   840
   tctcacaagg atcccaccag acaatttgac aaaggtgact attatgctca tccagaacac 900
   tacacccctg tttttcatga aagaattgct ccatatgcat ctgtcatcgt aaactgtatg 960
   tattgggaga agaggtttcc accattacta aatatggatc agttacagca attgatggag 1020
   actggttgtc ctttagtcgg cgtttgtgac ataacttgtg atattggagg ttccattgaa 1080
   tttatcaaca agagtacatc aatagagagg cctttctttc ggtatgatcc ttctaagaat 1140
   tcataccatg atgatatgga aggtgccgga gtggtctgct tggctgttga cattctccct 1200
   acagaattct ctaaagaggc ctcccaacat tttggaaaca tactatctag acttgttgct 1260
   agtttggcct cagtgaagca accggcagaa cttccttcct acttgagaag agcttgcatt 1320
   gcacatgctg gcagattaac tcctttgtat gaatatatcc ctaggatgag aaatactatg 1380
   atagatttgg cacccgcaaa aacaaatcca ttgcctgaca agaagtatag caccctggta 1440
   tctctcagtg ggcacctatt tgataagttc cttataaatg aagctttgga catcattgag 1500
   acagctggag gttcatttca cttggttaga tgtgaagttg gacaaagcac ggatgatatg 1560
   tcatactcag agcttgaagt aggagcagat gatactgcca cattggataa aattattgat 1620
   tccttgactt ctttagctaa tgaacatggt ggagatcacg atgccgggca agaaattgaa 1680
   ttagctctga agataggaaa agtcaatgag tatgaaactg acgtcacaat tgataaagga 1740
   gggccaaaga ttttaattct tggagctgga agagtctgtc ggccagctgc tgagtttctg 1800
   gcatcttacc cagacatatg tacctatggt gttgatgacc atgatgcaga tcaaattcat 1860
   gttatcgtgg catctttgta tcaaaaagat gcagaagaga cagttgatgg tattgaaaat 1920
   acaactgcta cccagcttga tgttgctgat attggaagcc tttcagatct tgtttctcag 1980
   gttgaggttg taattagctt gctgcctgct agttttcatg ctgccattgc aggagtatgc 2040
   atagagttga agaagcacat ggtaacggca agctatgttg atgaatccat gtcaaacttg 2100
   agccaagctg ccaaagatgc aggtgtaact atactttgtg aaatgggcct agatcctggc 2160
   atagatcact tgatgtcaat gaagatgatt gatgaagctc atgcacgaaa gggaaaaata 2220
   aaggcattta catcttactg tggtggattg ccatctccag ctgcagcaaa caatccgctt 2280
   gcctataaat tcagttggaa cccagctggt gcactccggt cagggaaaaa tcctgcagtc 2340
   tacaaatttc ttggtgagac gatccatgta gatggtcata acttgtatga atcagcaaag 2400
   aggctcagac tacgagagct tccagctttt gctctggaac acttgccaaa tcggaattcc 2460
   ttgatatatg gtgaccttta tggtatctcc aaagaagcat ccaccatata tagggctact 2520
   cttcgttacg aaggttttag tgagattatg gtaacccttt ccaaaactgg gttctttgat 2580
   gctgcaaatc atccactgct gcaagatact agtcgtccaa catataaggg tttccttgat 2640
   gaactactga ataatatctc cacaattaac acggacttag atattgaagc ctctggtgga 2700
   tacgatgatg acctgattgc cagactgttg aagctcgggt gttgcaaaaa taaggaaata 2760
   gctgttaaga cagtcaaaac catcaagttc ttgggactac atgaagagac tcaaatacct 2820
   aagggttgtt cgagcccatt tgatgtgatt tgccagcgaa tggaacagag gatggcctat 2880
   ggccacaatg agcaagacat ggtactgctc caccacgaag tcgaggtgga atacccggac 2940
   gggcaacccg ccgaaaagca ccaagcgacg ctactggagt tcgggaaggt tgaaaatggc 3000
   aggtccacca ctgccatggc gctgaccgtc ggcattccag cagcaatagg ggccctgcta 3060
   ttgctaaaga ataaggtcca gacgaaagga gtgatcaggc ctctgcaacc ggaaatctac 3120
   gttccagcat tggagatctt ggagtcgtcg ggcatcaagc tggttgagaa agtggagact 3180
   tga 3183
<210> 3
   <211> 1350
   <212> DNA
   <213> Escherichia coli
<400> 3
   atggcagaaa ttgttgtctc caaatttggc ggtaccagcg tagctgattt tgacgccatg
   60
   aaccgcagcg ctgatattgt gctttctgat gccaacgtgc gtttagttgt cctctcggct
   120
   tctgctggta tcactaatct gctggtcgct ttagctgaag gactggaacc tggcgagcga
   180
   ttcgaaaaac tcgacgctat ccgcaacatc cagtttgcca ttctggaacg tctgcgttac
   240
   ccgaacgtta tccgtgaaga gattgaacgt ctgctggaga acattactgt tctggcagaa
   300
   gcggcggcgc tggcaacgtc tccggcgctg acagatgagc tggtcagcca cggcgagctg
   360
   atgtcgaccc tgctgtttgt tgagatcctg cgcgaacgcg atgttcaggc acagtggttt
   420
   gatgtacgta aagtgatgcg taccaacgac cgatttggtc gtgcagagcc agatatagcc
   480
   gcgctggcgg aactggccgc gctgcagctg ctcccacgtc tcaatgaagg cttagtgatc
   540
   acccagggat ttatcggtag cgaaaataaa ggtcgtacaa cgacgcttgg ccgtggaggc
   600
   agcgattata cggcagcctt gctggcggag gctttacacg catctcgtgt tgatatctgg
   660
   accgacgtcc cgggcatcta caccaccgat ccacgcgtag tttccgcagc aaaacgcatt
   720
   gatgaaatcg cgtttgccga agcggcagag atggcaactt ttggtgcaaa agtactgcat
   780
   ccggcaacgt tgctacccgc agtacgcagc gatatcccgg tctttgtcgg ctccagcaaa
   840
   gacccacgcg caggtggtac gctggtgtgc aataaaactg aaaatccgcc gctgttccgc
   900
   gctctggcgc ttcgtcgcaa tcagactctg ctcactttgc acagcctgaa tatgctgcat
   960
   tctcgcggtt tcctcgcgga agttttcggc atcctcgcgc ggcataatat ttcggtagac
   1020
   ttaatcacca cgtcagaagt gagcgtggca ttaatccttg ataccaccgg ttcaacctcc
   1080
   actggcgata cgttgctgac gcaatctctg ctgatggagc tttccgcact gtgtcgggtg 1140
   gaggtggaag aaggtctggc gctggtcgcg ttgattggca atgacctgtc aaaagcctgc 1200
   ggcgttggca aagaggtatt cggcgtactg gaaccgttca acattcgcat gatttgttat 1260
   ggcgcatcca gccataacct gtgcttcctg gtgcccggcg aagatgccga gcaggtggtg 1320
   caaaaactgc atagtaattt gtttgagtaa 1350
<210> 4
   <211> 392
   <212> DNA
   <213> Zea mays
<400> 4
   gatctcgtca atttttccct tttgtaattt tgtaatttat aatttacttt cctgtatgct 60
   tctaactctg tcgttttaaa attttaataa aatttcagta ggggcttgcc cctcctgtcc 120
   tataaaaaaa agttgacgtg aatagatttc attaagaggt tggatgttag tgggatgaca 180
   tgactattag taggacgaga tgatgtggaa agttagtggg agatgatatg gatagttttt 240
   gctttcatcg aaaggttgga agttagtatg atgacatggc taatatagat acatagatat 300
   agactaccaa catggctgca tgcccccaag ctctcccact atatatatct ctggtagcac 360
   atcatcccaa ttcacaatgc ttacaaaaac cc 392
<210> 5
   <211> 903
   <212> DNA
   <213> Corynebacterium glutamicum
<400> 5
   ttatagaact ccagcttttt tcatgtcttc tcgcagagcc tcaagttcct gctcatttgg
   60
   agccattatt ggaagcctag gatctcctac gttgatgcct tgcagacgca gagcagcttt
   120
   tgccaagctg actccaccca agcgaccctg ggcagctacc agcggtgata gtttggcgtt
   180
   gatctccctc gcacggacca ggtcgccttc ctcgaaactt gtgtacaact cccttaatgc
   240
   tgtgggggct gcatgtccaa ttacggatat aaaacctgat ccgcccaaag caagccaaac
   300
   aaggtttagt gggtcatcgc ctgaatacca ggcaagtccc gtttctttga tcaatgacgt
   360
   ggctgcaacc aggtcccctt tggcgtcttt gaccgccaaa atcgtaggta attcactcag
   420
   gcgtctcatg gtatcagact caattggaat acctgaccta ccaggaatgt cgtagaggca
   480
   aattggcacc tctgttgctg ctgcgattgc cccgaagtgc gccagcaatc cctcctggct
   540
   cggcttactg taataaggag ttacgactaa aaggccgtct gcgccagcag aagcagcagc
   600
   ctccgcaagt tccacagatg tccgcgtgtt attggttccg acaccggcga ttagtttcgc
   660
   cctatcccct acttcctcac gcacggcctt gagcagttct agtttctcag cggcggttgt
   720
   cgttggggat tcaccagtgg tgcccgcgag caccaaagaa tccaagcctt tatcgactag
   780
   ataagccgcg acctcgcggc cagcagctat atcgatgtct ccactttccg tgaatggagt
   840
   taccattgct actcccacgg tgccaaagtg ctctactccg gtcttagctg ttaaacctgt
   900
   gct
   903
<210> 6
   <211> 171
   <212> DNA
   <213> Zea mays
<400> 6
   atggtttcgc cgacgaatct cctcccggcg cggaagatca cccctgtctc aaatggcggc 60
   gcagcgacgg cgagcccctc ttctccctcg gtggccgcac ggccacggcg actcccttca 120
   ggcctccaat ctgtgactgg tagagggaag gtttccttgg cagccatcac t 171
<210> 7
   <211> 300
   <212> PRT
   <213> Escherichia coli
<400> 7
   Ser Thr Gly Leu Thr Ala Lys Thr Gly Val Glu His Phe Gly Thr Val
   1               5                   10                  15
   Gly Val Ala Met Val Thr Pro Phe Thr Glu Ser Gly Asp Ile Asp Ile
               20                  25                  30
   Ala Ala Gly Arg Glu Val Ala Ala Tyr Leu Val Asp Lys Gly Leu Asp
           35                  40                  45
   Ser Leu Val Leu Ala Gly Thr Thr Gly Glu Ser Pro Thr Thr Thr Ala
       50                  55                  60
   Ala Glu Lys Leu Glu Leu Leu Lys Ala Val Arg Glu Glu Val Gly Asp
   65                  70                  75                  80
   Arg Ala Lys Leu Ile Ala Gly Val Gly Thr Asn Asn Thr Arg Thr Ser
                   85                  90                  95
   Val Glu Leu Ala Glu Ala Ala Ala Ser Ala Gly Ala Asp Gly Leu Leu
               100                 105                 110
   Val Val Thr Pro Tyr Tyr Ser Lys Pro Ser Gln Glu Gly Leu Leu Ala
           115                 120                 125
   His Phe Gly Ala Ile Ala Ala Ala Thr Glu Val Pro Ile Cys Leu Tyr
       130                 135                 140
   Asp Ile Pro Gly Arg Ser Gly Ile Pro Ile Glu Ser Asp Thr Met Arg
   145                 150                 155                 160
   Arg Leu Ser Glu Leu Pro Thr Ile Leu Ala Val Lys Asp Ala Lys Gly
                   165                 170                 175
   Asp Leu Val Ala Ala Thr Ser Leu Ile Lys Glu Thr Gly Leu Ala Trp
               180                 185                 190
   Tyr Ser Gly Asp Asp Pro Leu Asn Leu Val Trp Leu Ala Leu Gly Gly
           195                 200                 205
   Ser Gly Phe Ile Ser Val Ile Gly His Ala Ala Pro Thr Ala Leu Arg
       210                 215                 220
   Glu Leu Tyr Thr Ser Phe Glu Glu Gly Asp Leu Val Arg Ala Arg Glu
   225                 230                 235                 240
   Ile Asn Ala Lys Leu Ser Pro Leu Val Ala Ala Gln Gly Arg Leu Gly
                   245                 250                 255
   Gly Val Ser Leu Ala Lys Ala Ala Ser Arg Leu Gln Gly Ile Asn Val
               260                 265                 270
   Gly Asp Pro Arg Leu Pro Ile Met Ala Pro Asn Glu Gln Glu Leu Glu
           275                 280                 285
   Ala Leu Arg Glu Asp Met Lys Lys Ala Gly Val Leu
       290                 295                 300
<210> 8
   <211> 449
   <212> PRT
   <213> Escherichia coli
<400> 8
   Met Ala Glu Ile Val Val Ser Lys Phe Gly Gly Thr Ser Val Ala Asp
   1 5 10 15
   Phe Asp Ala Met Asn Arg Ser Ala Asp Ile Val Leu Ser Asp Ala Asn
   20 25 30
   Val Arg Leu Val Val Leu Ser Ala Ser Ala Gly Ile Thr Asn Leu Leu
   35 40 45
   Val Ala Leu Ala Glu Gly Leu Glu Pro Gly Glu Arg Phe Glu Lys Leu
   50 55 60
   Asp Ala Ile Arg Asn Ile Gln Phe Ala Ile Leu Glu Arg Leu Arg Tyr
   65 70 75 80
   Pro Asn Val Ile Arg Glu Glu Ile Glu Arg Leu Leu Glu Asn Ile Thr
   85 90 95
   Val Leu Ala Glu Ala Ala Ala Leu Ala Thr Ser Pro Ala Leu Thr Asp
   100 105 110
   Glu Leu Val Ser His Gly Glu Leu Met Ser Thr Leu Leu Phe Val Glu
   115 120 125
   Ile Leu Arg Glu Arg Asp Val Gln Ala Gln Trp Phe Asp Val Arg Lys
   130 135 140
   Val Met Arg Thr Asn Asp Arg Phe Gly Arg Ala Glu Pro Asp Ile Ala
   145 150 155 160
   Ala Leu Ala Glu Leu Ala Ala Leu Gln Leu Leu Pro Arg Leu Asn Glu
   165 170 175
   Gly Leu Val Ile Thr Gln Gly Phe Ile Gly Ser Glu Asn Lys Gly Arg
   180 185 190
   Thr Thr Thr Leu Gly Arg Gly Gly Ser Asp Tyr Thr Ala Ala Leu Leu
   195 200 205
   Ala Glu Ala Leu His Ala Ser Arg Val Asp Ile Trp Thr Asp Val Pro
   210 215 220
   Gly Ile Tyr Thr Thr Asp Pro Arg Val Val Ser Ala Ala Lys Arg Ile
   225 230 235 240
   Asp Glu Ile Ala Phe Ala Glu Ala Ala Glu Met Ala Thr Phe Gly Ala
   245 250 255
   Lys Val Leu His Pro Ala Thr Leu Leu Pro Ala Val Arg Ser Asp Ile
   260 265 270
   Pro Val Phe Val Gly Ser Ser Lys Asp Pro Arg Ala Gly Gly Thr Leu
   275 280 285
   Val Cys Asn Lys Thr Glu Asn Pro Pro Leu Phe Arg Ala Leu Ala Leu
       290                 295                 300
   Arg Arg Asn Gln Thr Leu Leu Thr Leu His Ser Leu Asn Met Leu His
   305                 310                 315                 320
   Ser Arg Gly Phe Leu Ala Glu Val Phe Gly Ile Leu Ala Arg His Asn
                   325                 330                 335
   Ile Ser Val Asp Leu Ile Thr Thr Ser Glu Val Ser Val Ala Leu Thr
               340                 345                 350
   Leu Asp Thr Thr Gly Ser Thr Ser Thr Gly Asp Thr Leu Leu Thr Gln
           355                 360                 365
   Ser Leu Leu Met Glu Leu Ser Ala Leu Cys Arg Val Glu Val Glu Glu
       370                 375                 380
   Gly Leu Ala Leu Val Ala Leu Ile Gly Asn Asp Leu Ser Lys Ala Cys
   385                 390                 395                 400
   Ala Val Gly Lys Glu Val Phe Gly Val Leu Glu Pro Phe Asn Ile Arg
                   405                 410                 415
   Met Ile Cys Tyr Gly Ala Ser Ser His Asn Leu Cys Phe Leu Val Pro
               420                 425                 430
   Gly Glu Asp Ala Glu Gln Val Val Gln Lys Leu His Ser Asn Leu Phe
           435                 440                 445
   Glu
<210> 9
   <211> 1155
   <212> DNA
   <213> Zea mays
<400> 9
   ccgatttggc aagtgttcca gagcaaaagc tggaagctct cgtagtctga gcctctttgc 60
   tgattcatac aagttatgac catctacatg gatcgtctca ccaagaaatt tgtagactgc 120
   aggatttttc cctgaccgga gtgcaccagc tgggttccaa ctgaatttat aggcaagcgg 180
   attgtttgct gcagctggag atggcaatcc accacagtaa gatgtaaatg cctttatttt 240
   tccctttcgt gcatgagctt catcaatcat cttcattgac atcaagtgat ctatgccagg 300
   atctaggccc atttcacaaa gtatagttac acctgcatct ttggcagctt ggctcaagtt 360
   tgacatggat tcatcaacat agcttgccgt taccatgtgc ttcttcaact ctatgcatac
   420
   tcctgcaatg gcagcatgaa aactagcagg cagcaagcta attacaacct caacctgaga
   480
   aacaagatct gaaaggcttc caatatcagc aacatcaagc tgggtagcag ttgtattttc
   540
   aataccatca actgtctctt ctgcatcttt ttgatacaaa gatgccacga taacatgaat
   600
   ttgatctgca tcatggtcat caacaccata ggtacatatg tctgggtaag atgccagaaa
   660
   ctcagcagct ggccgacaga ctcttccagc tccaaaccgg tgctgcctgc tagttttcat
   720
   gctgccattg caggagtatg catagagttg aagaagcaca tggtaacggc aagctatgtt
   780
   gatgaatcca tgtcaaactt gagccaagct gccaaagatg caggtgtaac tatactttgt
   840
   gaaatgggcc tagatcctgg catagatcac ttgatgtcaa tgaagatgat tgatgaagct
   900
   catgcacgaa agggaaaaat aaaggcattt acatcttact gtggtggatt gccatctcca
   960
   gctgcagcaa acaatccgct tgcctataaa ttcagttgga acccagctgg tgcactccgg
   1020
   tcagggaaaa atcctgcagt ctacaaattt cttggtgaga cgatccatgt agatggtcat
   1080
   aacttgtatg aatcagcaaa gaggctcaga ctacgagagc ttccagcttt tgctctggaa
   1140
   cacttgccaa atcgg
   1155
<210> 10
   <211> 1967
   <212> DNA
   <213> Artificial Sequence
<220>
   <223> Chimeric DNA molecule of a maize hsp70 intron and a LKR/SDH gene suppression molecule
<400> 10
   accgtcttcg gtacgcgctc actccgccct ctgcctttgt tactgccacg tttctctgaa 60
   tgctctcttg tgtggtgatt gctgagagtg gtttagctgg atctagaatt acactctgaa 120
   atcgtgttct gcctgtgctg attacttgcc gtcctttgta gcagcaaaat atagggacat 180
   ggtagtacga aacgaagata gaacctacac agcaatacga gaaatgtgta atttggtgct 240
   tagcggtatt tatttaagca catgttggtg ttatagggca cttggattca gaagtttgct 300
   gttaatttag gcacaggctt catactacat gggtcaatag tatagggatt catattatag 360
   gcgatactat aataatttgt tcgtctgcag agcttattat ttgccaaaat tagatattcc 420
   tattctgttt ttgtttgtgt gctgttaaat tgttgatccc gatttggcaa gtgttccaga 480
   gcaaaagctg gaagctctcg tagtctgagc ctctttgctg attcatacaa gttatgacca 540
   tctacatgga tcgtctcacc aagaaatttg tagactgcag gatttttccc tgaccggagt 600
   gcaccagctg ggttccaact gaatttatag gcaagcggat tgtttgctgc agctggagat 660
   ggcaatccac cacagtaaga tgtaaatgcc tttatttttc cctttcgtgc atgagcttca 720
   tcaatcatct tcattgacat caagtgatct atgccaggat ctaggcccat ttcacaaagt 780
   atagttacac ctgcatcttt ggcagcttgg ctcaagtttg acatggattc atcaacatag 840
   cttgccgtta ccatgtgctt cttcaactct atgcatactc ctgcaatggc agcatgaaaa 900
   ctagcaggca gcaagctaat tacaacctca acctgagaaa caagatctga aaggcttcca 960
   atatcagcaa catcaagctg ggtagcagtt gtattttcaa taccatcaac tgtctcttct 1020
   gcatcttttt gatacaaaga tgccacgata acatgaattt gatctgcatc atggtcatca 1080
   acaccatagg tacatatgtc tgggtaagat gccagaaact cagcagctgg ccgacagact 1140
   cttccagctc caaaccggtg ctgcctgcta gttttcatgc tgccattgca ggagtatgca 1200
   tagagttgaa gaagcacatg gtaacggcaa gctatgttga tgaatccatg tcaaacttga 1260
   gccaagctgc caaagatgca ggtgtaacta tactttgtga aatgggccta gatcctggca 1320
   tagatcactt gatgtcaatg aagatgattg atgaagctca tgcacgaaag ggaaaaataa 1380
   aggcatttac atcttactgt ggtggattgc catctccagc tgcagcaaac aatccgcttg
   1440
   cctataaatt cagttggaac ccagctggtg cactccggtc agggaaaaat cctgcagtct
   1500
   acaaatttct tggtgagacg atccatgtag atggtcataa cttgtatgaa tcagcaaaga
   1560
   ggctcagact acgagagctt ccagcttttg ctctggaaca cttgccaaat cggaattaac
   1620
   gcctgaagga ataaatataa atgacgaaat tttgatgttt atctctgctc ctttattgtg
   1680
   accataagtc aagatcagat gcacttgttt taaatattgt tgtctgaaga aataagtact
   1740
   gacagtattt tgatgcattg atctgcttgt ttgttgtaac aaaatttaaa aataaagagt
   1800
   ttcctttttg ttgctctcct tacctcctga tggtatctag tatctaccaa ctgacactat
   1860
   attgcttctc tttacatacg tatcttgctc gatgccttct ccctagtgtt gaccagtgtt
   1920
   actcacatag tctttgctca tttcattgta atgcagatac caagcgg
   1967
<210> 11
   <211> 1350
   <212> DNA
   <213> Escherichia coli
<400> 11
   atggcagaaa ttgttgtctc caaatttggc ggtaccagcg tagctgattt tgacgccatg 60
   aaccgcagcg ctgatattgt gctttctgat gccaacgtgc gtttagttgt cctctcggct 120
   tctgctggta tcactaatct gctggtcgct ttagctgaag gactggaacc tggcgagcga 180
   ttcgaaaaac tcgacgctat ccgcaacatc cagtttgcca ttctggaacg tctgcgttac 240
   ccgaacgtta tccgtgaaga gattgaacgt ctgctggaga acattactgt tctggcagaa 300
   gcggcggcgc tggcaacgtc tccggcgctg acagatgagc tggtcagcca cggcgagctg 360
   atgtcgaccc tgctgtttgt tgagatcctg cgcgaacgcg atgttcaggc acagtggttt 420
   gatgtacgta aagtgatgcg taccaacgac cgatttggtc gtgcagagcc agatatagcc
   480
   gcgctggcgg aactggccgc gctgcagctg ctcccacgtc tcaatgaagg cttagtgatc
   540
   acccagggat ttatcggtag cgaaaataaa ggtcgtacaa cgacgcttgg ccgtggaggc
   600
   agcgattata cggcagcctt gctggcggag gctttacacg catctcgtgt tgatatctgg
   660
   accgacgtcc cgggcatcta caccaccgat ccacgcgtag tttccgcagc aaaacgcatt
   720
   gatgaaatcg cgtttgccga agcggcagag atggcaactt ttggtgcaaa agtactgcat
   780
   ccggcaacgt tgctacccgc agtacgcagc gatatcccgg tctttgtcgg ctccagcaaa
   840
   gacccacgcg caggtggtac gctggtgtgc aataaaactg aaaatccgcc gctgttccgc
   900
   gctctggcgc ttcgtcgcaa tcagactctg ctcactttgc acagcctgaa tatgctgcat
   960
   tctcgcggtt tcctcgcgga agttttcggc atcctcgcgc ggcataatat ttcggtagac
   1020
   ttaatcacca cgtcagaagt gagcgtggca ttaatccttg ataccaccgg ttcaacctcc
   1080
   actggcgata cgttgctgac gcaatctctg ctgatggagc tttccgcact gtgtcgggtg
   1140
   gaggtggaag aaggtctggc gctggtcgcg ttgattggca atgacctgtc aaaagcctgc
   1200
   ggcgttggca aagaggtatt cggcgtactg gaaccgttca acattcgcat gatttgttat
   1260
   ggcgcatcca gccataacct gtgcttcctg gtgcccggcg aagatgccga gcaggtggtg
   1320
   caaaaactgc atagtaattt gtttgagtaa
   1350

## Description

### BACKGROUND OF THE INVENTION

### 1. Field of the Invention

Disclosed herein are DNA constructs useful for producing transgenic plants with parts having increased lysine content and methods of using such DNA constructs for producing transgenic plants and seed, harvested materials from the plants, and meal and food products prepared from such. Such DNA constructs are useful for producing transgenic plants with improved nutritional benefit provided by increased accumulation of lysine in the seed. Also disclosed are polynucleotide molecules and methods useful for producing transgenic plants with increased lysine content in their seed.

### 2. Description of the Related Art

*Zea mays,* commonly known as maize or corn, is a grain widely used as animal feed and human food. The grain, herein referred to as a kernel or seed, is a source of protein, starch and oil for many lower animals including swine, beef and dairy cattle, fish and poultry. In some countries, such as Mexico, over 70% of the harvested corn is used for human consumption, and corn and corn-derived products such as hominy and tortillas are dietary staples. In the kernel, the bulk of the amino acid composition is determined by the amount and type of amino acids contained in polypeptides. Only a relatively small portion, up to 10%, of the available amino acids in the kernel exists as free amino acid pools; the rest are contained in various proteins. In corn, most of the polypeptides found in the kernel are seed storage proteins or zeins. These seed storage proteins are synthesized as the kernel develops and are used as a source of energy as the kernel germinates and begins to grow. The seed storage proteins, however, contain little to no lysine. Of the ten amino acids deemed essential in a mixed grain feed (arginine, histidine, isoleucine, leucine, lysine, methionine or cysteine, phenylalanine or tyrosine, threonine, tryptophan, and valine), corn is particularly lacking not only in lysine, but also in threonine and methionine. The lack of these essential amino acids, especially lysine, requires that feed corn be supplemented with these nutrients, often provided by the addition of soybean meal or synthetic lysine. It would be of benefit to the art to increase the level of lysine in corn seed as a means of making the seed more nutritious as a food or feed grain. Additional benefit is realized if the grain requires little or no supplementation with additional lysine.

In human nutrition, the poor tend to consume diets that are relatively high in inexpensive starchy foods and relatively low in high quality protein. Kwashiorkor is a form of malnutrition caused by inadequate quality protein intake in the presence of fair to good energy (total calories) intake. Early symptoms are very general and include fatigue, irritability, and lethargy. As protein deficiency continues, growth failure, loss of muscle mass, generalized swelling (edema), and decreased immunity occur. A large, protuberant belly is common. Skin conditions (such as dermatitis, changes in pigmentation, thinning of hair, and vitiligo) are seen frequently. Shock and coma precede death. One government estimate suggests that as many as 50% of elderly persons in nursing homes in the U.S. suffer from protein-calorie malnutrition. Thus, corn products with improved protein quality due to higher levels of lysine would significantly improve not only the nutritional quality of the diet but the overall general level of health.

A plant molecular biology approach to increasing lysine levels involves the identification and introduction of genes into corn to affect the levels of lysine in the kernel without negatively affecting agronomic properties. Lysine is derived from aspartate, the metabolism of which is interconnected with pathways involving threonine, methionine and isoleucine. Lysine itself serves as an end product to modify and regulate enzymes in the pathway for its own production. Key enzymes involved in lysine metabolism are aspartate kinase (AK) and dihydrodipicolinic acid synthase (DHDPS), while lysine-ketoglutarate reductase (LKR, a bifunctional enzyme also known as saccharopine dehydrogenase, SDH) appears to play a key role in lysine catabolism. AK and DHDPS are feedback regulated by lysine; as lysine levels increase, the amino acid down-regulates the activity of these enzymes.

Thus, in order to increase levels of lysine, it would be beneficial to have a feedback insensitive version of AK or DHDPS. Lysine-insensitive variants of a bacterial AK gene have been described as well as several variants from plants (see Falco et al., U.S. Patent No. 5,773,691). Lysine-insensitive forms of AK have been identified from barley, corn, and tobacco. A bacterial DHDPS gene, isolated from *E*. *coli,* has been shown to be at least 20-fold less sensitive to increases in lysine levels (Glassman, et al., U.S. Patent No. 5,258,300; Galili, et al., U.S. Patent No. 5,367,110).

Falco, et al. (U.S Patent No. 5,773,691 and 6,459,019, U.S. Patent Application Publication Nos. U.S. 2003/0056242), and Dizigan et al. (U.S. Patent Application Publication No. 2005/0132437), describe the isolation and use of lysine feedback-insensitive aspartate kinase (AK, the gene known as *lysC*) from *E*. *coli,* DHDPS from *E. coli,* and DHDPS from *Corynebacterium* (known herein as *CORgl.dapA*) to generate transgenic rapeseed, tobacco, corn and soybean plants with increased levels of lysine in seed.

The present invention provides DNA constructs that when expressed in plant cells provides an increased nutritional component of a plant product, in particular, increased lysine in a plant seed.

### SUMMARY OF THE INVENTION

Provided herein are DNA constructs that when operative in the genome of a transgenic plant confer increased levels of lysine in the seed of the plant. The harvested high lysine seeds are useful for the production of food and feed products, such as corn flour (also known as "masa harina"), corn meal and meal products that require little or no lysine supplementation. Preferably, the transgenic plant and seed is a crop plant and seed, preferably a monocot plant and seed, and most preferably, a corn plant and seed.

The invention provides transgenic plants and seed that comprise the DNA constructs and have increased levels of lysine in the seed. The DNA constructs of the present invention comprise a molecule that when expressed in corn cells inhibits the production of a lysine degradation polypeptide and a molecule that when expressed in corn cells provides for production of a lysine insensitive dihydrodipicolinic acid synthase polypeptide and a lysine insensitive aspartate kinase.

The harvested grain, even when nonviable and therefore unusable as seed, nevertheless offers the higher levels of lysine and higher protein quality enabled by this invention. Such harvested grain is capable of being processed in the same manner as conventional grain. Accordingly, in another embodiment, the invention provides a high lysine animal feed product prepared by growing a transgenic plant of the invention and harvesting the high lysine containing seed, or by processing a seed of the invention or portion thereof. In yet another embodiment, the invention provides a meal or meal product prepared by growing a transgenic plant of the invention and harvesting the high lysine containing seed, or by processing a seed of the invention or portion thereof.

In one embodiment of the invention, a DNA construct comprises novel polynucleotide compositions that are enhanced for expression in plant cells, especially corn cells. One of the novel polynucleotides (SEQ ID NO:5) that encodes a dihydrodipicolinic acid synthase polypeptide, and another novel polynucleotide (SEQ ID NO:9) that is homologous and complementary to a portion of a lysine ketoglutarate reductase polynucleotide coding sequence. The DNA construct when expressed in a plant cell, especially a corn plant cell and preferably a corn seed cell causes an increase in lysine in the corn seed. The DNA construct comprises polynucleotides for example, a sequence such as SEQ ID NO:1 that encodes a lysine insensitive dihydrodipicolinic acid synthase, a sequence such as SEQ ID NO:2 that encodes a lysine ketoglutarate reductase that is used as a template for designing inhibitory RNAs, and a sequence such as SEQ ID NO:3 that encodes an aspartate kinase. The DNA construct preferably comprises a promoter molecule that is enhanced for directing the transcription of an operably linked polynucleotide in a corn seed. Additionally, a novel DNA molecule (SEQ ID NO:10) that comprises an gene suppression molecule with a lysine ketoglutarate reductase gene target in which the gene suppression molecule is embedded within an intron of the DNA construct.

In another embodiment, the application provides a method of making a high lysine corn seed by crossing inbred parents to provide a hybrid seed, wherein each parent contains one or more plant expression cassettes of the DNA constructs of the present invention in its genome and the expression cassettes are different from each other in each parent and neither parent contains all three, and harvesting the seed that has a lysine content higher than either of the parents.

In another embodiment, the invention provides a method of making a high lysine human food or animal feed product comprising the step of processing a seed or a portion thereof from a transgenic plant having a genome comprising an exogenous DNA construct or collection of constructs according to the present invention. Additionally, the invention provides a method of making a high lysine human food or animal feed product comprising the steps of growing a transgenic plant to produce 4 seed, the seed having a genome comprising an exogenous DNA construct of the present invention, and harvesting the seed.

### BRIEF DESCRIPTION OF THE DRAWINGS

FIG. 1. Plasmid map illustrating pMON66649
FIG. 2. Plasmid map illustrating pMON80003
FIG. 3. Plasmid map illustrating pMON79465
FIG. 4. Plasmid map illustrating pMON93092
FIG. 5. Plasmid map illustrating pMON93093
FIG. 6. Plasmid map illustrating pMON80378
FIG. 7. Free lysine content of R2 kernels from events comprising pMON93092.
FIG. 8. Free lysine content of R2 kernels from events comprising pMON93093.

### DETAILED DESCRIPTION OF THE INVENTION

A transgenic plant or seed that shows a desired trait, for example, increased lysine of the present invention, comprises a particular exogenous DNA inserted into the genome of the transgenic plant that imparts the desired trait. The trait is expressed as a measurable change from the naturally occurring trait in a control plant, for example, a plant or seed of substantially the same genotype that lacks that particular exogenous DNA. Preferably, the change is measured by comparing the expression of the trait in a transgenic plant or seed having the particular exogenous DNA associated with the desired trait to the expression of the same trait in a control plant or seed. "High lysine maize" is therefore a corn (maize) plant with increased lysine in any plant part, preferably a seed, which may also be referred to herein as a kernel or in the aggregate as grain. The present invention provides DNA constructs and seeds that contain at least one of the plant expression cassettes of the DNA constructs of the present inventions in its genome, wherein the seed has a higher lysine content than seeds not containing the construct.

Increased lysine may be exhibited by the plant by accumulation of increased amounts of the amino acid in the kernel and may be measured by any suitable method, such as mass spectrophotometry or high performance liquid chromatography of appropriately extracted tissue. A transgenic corn kernel of the present invention with increased lysine is especially useful as a feed or food product, a meal or meal product, a source of purified corn protein products, or a source of other products processed from the kernel that contain a higher lysine content than nontransgenic kernels of a similar variety.

Corn grain with a level of approximately 4000 parts per million (ppm) of total lysine has a sufficient quantity of lysine such that the livestock feed or food product made from the grain does not require supplementation from additional lysine sources. The high lysine corn of the present invention achieves this level of lysine production and accumulation in a corn kernel. The present invention is a substantial improvement over previously described methods and compositions for increasing lysine in a corn kernel (for example, U.S. Patent No. 5,773,691).

Efforts to increase amino acid levels in transgenic plants include expressing recombinant DNA molecules that encode proteins in an amino acid synthesis pathway at higher levels than native genes. One method for producing enhanced levels of lysine in corn is by expression of a bacterial dihydrodipicolinic acid synthase (DHDPS) as disclosed in U.S. Patents 5,288,300, 6,459,019, and U.S. Patent Application Publication 2003/0056242 A1 and suppression of endogenous LKR (U.S. Patent Application Publication 2005/0193444).

Any of the plants or parts thereof of the present invention may be processed to produce a feed, flour, meal or protein preparation. A particularly preferred plant part for this purpose is the seed. In one preferred embodiment the feed, flour, meal or protein preparation is designed for use in feeding farm animals (livestock). Methods to produce feed, flour, meal and protein preparations are known in the art, for example, U.S. Patents 4,957,748; 5,100,679; 5,219,596; 5,936,069; 6,005,076; 6,146,669; and 6,156,227. In a preferred embodiment, the protein preparation is a high protein preparation, such as a concentrate or isolate. The high protein preparation preferably has a protein content of greater than 5% w/v, more preferably 10% w/v, and even more preferably 15% w/v.

In a further embodiment, meal of the present invention may be blended with other meals. In a preferred embodiment, the meal produced from plants or seeds of the present invention or generated by a method of the present invention constitutes greater than about 0.5%, about 1 %, about 5%, about 10%, about 25%, about 50%, about 75% or about 90% by volume or weight of the meal component of any product. In another embodiment, the meal preparation may be blended and can constitute greater than about 10%, about 25%, about 35%, about 50% or about 75% of the blend by volume. The present invention also provides a method of enhancing the growth of a human or lower animal comprising feeding the human or lower animal a diet wherein at least a portion of the diet is a high lysine corn comprising one or more of the plant expression cassettes of the present invention. As used herein, the term "lower animal" is intended to encompass the feeding of avians and fish as well as mammals.

### Recombinant DNA Constructs

The present invention provides DNA constructs that comprise operably linked polynucleotides that are effective for imparting increased lysine in corn kernels. The term "DNA construct" includes, but is not limited to the polynucleotide molecules that direct the transcription of linked polynucleotide molecules in a plant cell. At least a portion of a DNA construct of the present invention is used in a method to transform a plant cell, that is, the portion that is stably inserted into the genome of the cell. The portion provides for the high lysine phenotype in the corn kernels or meal produced from those kernels and is an aspect of the present invention. A deposit by Monsanto Technology LLC of DNA constructs pMON93092 and pMON09093 has been made under the Budapest Treaty with the American Type Culture Collection (ATCC) as accessions numbered PTA-6733 and PTA-6734, respectively. These deposits form a part of the disclosure of this invention, and while the sequences provided herein in the written disclosure are believed to be accurate, the polynucleotide sequence of the DNA molecules that comprise regulatory elements, coding molecules and noncoding molecules can be determined from the deposit if necessary and used to correct those sequences disclosed herein.

The polynucleotide molecules are linked in recombinant DNA constructs using methods known to those of ordinary skill in the art. An example of a useful technology for building DNA constructs is the GATEWAY™ cloning technology (Invitrogen Life Technologies, Carlsbad, California) that uses the site-specific recombinase LR cloning reaction of the Integrase/att system from bacteriophage lambda for vector construction instead of restriction endonucleases and ligases. The GATEWAY™ Cloning Technology Instruction Manual supplied by Invitrogen also provides concise directions for routine cloning of any desired polynucleotide into a vector comprising operable plant expression elements. Additional methods are disclosed in Molecular Cloning: A Laboratory Manual, 3rd edition Volumes 1, 2, and 3. J.F. Sambrook, D.W. Russell, and N. Irwin, Cold Spring Harbor Laboratory Press, 2000 (referred to herein as Sambrook, *et al*.).

As used herein "exogenous DNA or isolated DNA" refers to DNA molecule that is not normally found in the genome of a host cell, or a DNA not normally found in the host genome in an identical context, or any two sequences adjacent to each other that are not normally or naturally adjacent to each other. Exogenous DNA may include a DNA or RNA polynucleotide molecule native to the host genome or may comprise the native polynucleotide altered by the addition or deletion of one or more polynucleotides or modifications to associated polynucleotide regulatory elements or other polynucleotide sequences as discussed herein. In addition to regulatory elements, an exogenous DNA may contain a coding sequence that encodes a protein or polypeptide, or a noncoding sequence that produces a non-protein product, such as an RNA molecule that affects the transcription or translation of another DNA sequence. A DNA construct of the present invention comprising a coding sequence and a noncoding sequence.

Exogenous DNA constructs used for transforming plant cells will comprise the coding sequence of interest and usually other regulatory elements as discussed herein such as, but not limited to promoters, introns, 5' and 3' untranslated regions, leader sequences, localization and transit sequences and enhancers, the operable linkage of these regulatory elements to express an RNA in a plant cell being referred to herein as a heterologous plant expression cassette. The DNA constructs that are listed in Table 1 illustrate the linkage of regulatory elements in expression cassettes that express coding sequences and non-coding sequences, and the average lysine levels measured in transgenic maize seed comprising each construct. A promoter DNA molecule of an exogenous DNA construct of the present invention is exemplified by a seed-enhanced or seed-specific promoter that include, for example those for use in a monocot crop plant, such as, a maize L3 promoter (U.S. Patent 6,433,252 or homologs thereof, and referred to as P-Zm.L3 in the present invention), a maize B32 promoter (SEQ ID NO:4 or homologs thereof, herein referred to as P-Zm.B32), a maize gamma coixin promoter (U.S. Patent 6,635,806, or homologs thereof, herein referred to as P-Zm.Gcx), or anyone of which is used in a DNA construct of the present invention or other known promoters that provide for enhanced transcription in a corn seed is herein referred to as P-Zm.seed. Examples of additional promoters that function in seeds, include but are not limited to promoters that drive expression of seed storage proteins, such as, rice glutelin and the prolamin proteins of *Triticeae* species and promoters that control the expression of enzymes involved in starch synthesis in the seed, such as, promoters that drive expression of monocot ADP glucose starch glycosyl transferase. The selected seed promoter is operably linked to a DHDPS coding region (for example, SEQ ID NO:1, herein referred to as CORgl.dapA) or an aspartate kinase coding region (for example, SEQ ID NO:3). Additional seed-enhanced and seed-specific promoters are known in the art and can be selected and tested in operable linkage with the DNA coding molecules and non-coding molecules of the present invention by methods described in the present invention or similar methods known to those skilled in the art of plant molecular biology. A seed enhanced promoter primarily expresses in all of or in a part of a seed, although it may secondarily express in other plant cells, tissues or organs of the plant. A part of a seed includes, but is not limited to, an embryo, an endosperm, a seed coat, a coleoptile, a cotyledon, a hypocotyl, an aleurone layer, a pericarp, or a scutellum. Additional regulatory regions provided in the DNA constructs can include, but are not limited to, an intron (I-), a transit signal (TS-), a 5' untranslated leader (L-), a 3' polyadenylation region (T-), or a non-coding antisense region. A selectable or scorable marker expression cassette can also be included to assist in selection or identification of transformed plant cells and tissues. The construct of the present invention optionally contains an expression cassette that provides glyphosate tolerance to the corn plant and can be used as a selectable marker. As used herein "transgene" means an exogenous DNA that is incorporated into a host genome or is capable of autonomous replication in a host cell and is capable of causing the expression of one or more cellular products. Exemplary transgenes will provide the host cell, plants regenerated therefrom or parts of those plants, with a novel phenotype relative to the corresponding non-transformed cell or plant, for example, increased lysine in a corn kernel. Transgenes may be directly introduced into a plant by genetic transformation, and are preferably stable and heritable so that they may be inherited from a plant of any previous generation that was transformed with the exogenous DNA.

As used herein, "gene" or "coding sequence" means a DNA molecule from which an RNA molecule is transcribed. The DNA molecule provides a coding region (CR-) for an mRNA that encodes a protein product, or an RNA that functions as a gene suppression molecule, or a structural RNA molecule such as a tRNA, rRNA, snRNA, or other RNA. As used herein "expression" refers to the combination of intracellular processes, including transcription and translation, by which a DNA molecule, such as a gene, produces a polypeptide or an RNA molecule. An exemplary coding sequence is a *Corynebacterium* dihydrodipicolinate synthase gene (DHDPS; Bonnassie *et al.,* 1990; Richaud *et al.,* 1986).useful for the production of corn kernels with increased lysine. A corn plant, transformed to contain and express a *Corynebacterium* DHDPS gene, or any other gene resulting in increased lysine in kernel tissue, is also referred to as a high lysine corn plant. The *Corynebacterium* DHDPS coding region of the present invention is additionally modified to provide enhanced expression in a plant cell, preferably a monocot cell and most preferably a corn cell. The modified *Corynebacterium* DHDPS coding region used in the present invention is disclosed as SEQ ID NO:5, herein referred to as CORgl.dapA.nno.

As used herein "promoter" means a region of DNA sequence that initiates transcription of RNA from DNA. Promoters are located upstream of DNA to be transcribed and have regions that act as binding sites for RNA polymerase and have regions that work with other factors to promote RNA transcription. More specifically, basal promoters in plants comprise canonical regions associated with the initiation of transcription, such as CAAT and TATA boxes. In the present invention, preferred promoter molecules and 5' UTR molecules allow for transcription in seed cells or tissues at a rate or level greater than in other cells and tissues of the plant. Promoter molecules can be selected from those know in the art for example, promoters are described in U.S. Patent 6,437,217 (maize RS81 promoter), U.S. Patent 5,641,876 (rice actin promoter), U.S. Patent 6,426,446 (maize RS324 promoter), U.S. Patent 6,429,362 (maize PR-1 promoter), U.S. Patent 6,232,526 (maize A3 promoter), U.S. Patent 6,177,611 (constitutive maize promoters), U.S. Patents 5,322,938, 5,352,605, 5,359,142 and 5,530,196 (35S promoter), U.S. Patent 6,433,252 (maize L3 oleosin promoter, P-Zm.L3), U.S. Patent 6,429,357 (rice actin 2 promoter as well as a rice actin 2 intron), U.S. Patent 5,837,848 (root specific promoter), U.S. Patent 6,294,714 (light inducible promoters), U.S. Patent 6,140,078 (salt inducible promoters), U.S. Patent 6,252,138 (pathogen inducible promoters), U.S. Patent 6,175,060 (phosphorus deficiency inducible promoters), U.S. Patent 6,635,806 (gamma-coixin promoter, P-Cl.Gcx), and U.S. patent application Serial No. 09/757,089 (maize chloroplast aldolase promoter). Additionally, chimeric promoter molecules can be constructed that have cis elements derived from heterologous sources that provide further enhancement of promoter expression profile useful in the present invention. For example, plant virus promoter elements have been shown to provide enhancements to plant promoter elements when operably linked (U.S. Patent 6,660,911).

DNA constructs for use in transforming plants typically also comprise other regulatory elements in addition to a promoter, such as but not limited to 3' untranslated regions (containing polyadenylation sites), transit or signal peptides for targeting a protein to a plant cellular organelle, particularly to a chloroplast, leucoplast or other plastid organelle.

One skilled in the art would know various introns, enhancers, transit peptides, targeting signal sequences, 5' and 3' untranslated regions (UTRs) useful in the design of effective plant expression vectors, such as those disclosed, for example, in U.S.

Patent Application Publication 2003/01403641.

A 5' UTR that functions as a translation leader sequence is a DNA genetic element located between the promoter sequence of a gene and the coding sequence. The translation leader sequence is present in the fully processed mRNA upstream of the translation start sequence. The translation leader sequence may affect processing of the primary transcript to mRNA, or it may affect mRNA stability or translation efficiency. Examples of translation leader sequences include maize and petunia heat shock protein leaders (U.S. Patent No. 5,362,865), plant virus coat protein leaders, plant Rubisco leaders, among others (Turner and Foster, 1995). Leader molecules associated in the native context with promoter molecules described in the present invention include for example, the maize B32 leader (L-Zm.B32), the rice actin 1 leader (L-Os.Act1), the gamma coixin leader (L-Cl.Gcx)..

The 3' untranslated region (3' UTR) or 3' polyadenylation region means a DNA molecule linked to and located downstream of a structural polynucleotide molecule and includes polynucleotides that provide a polyadenylation signal and other regulatory signals capable of affecting transcription, mRNA processing or gene expression. The polyadenylation signal functions in plants to cause the addition of polyadenylate nucleotides to the 3' end of the mRNA precursor. The polyadenylation sequence can be derived from the natural gene, from a variety of plant genes, or from *Agrobacterium* T-DNA genes. Examples of 3' UTR regions are the nopaline synthase 3' region (nos 3'; Fraley *et al.,* 1983), wheat hsp17 (T-Ta.Hsp17), and T-Ps.RbcS2:E9 (pea rubisco small subunit), those disclosed in WO0011200A2 and other 3' UTRs known in the art can be tested and used in combination with a DHDPS or AK coding region, herein referred to as T-3'UTR.

Transit signals generally refer to peptide molecules that when linked to a protein of interest directs the protein to a particular tissue, cell, subcellular location, or cell organelle. Examples include, but are not limited to, chloroplast transit peptides, nuclear targeting signals, and vacuolar signals. The plastid transit peptide is of particular utility in the present invention to direct expression of the DHDPS enzyme to the plastids in a seed. A chloroplast transit peptide (CTP) can be engineered to be fused to the N terminus of proteins that are to be targeted into the plant chloroplast. Many chloroplast-localized proteins are expressed from nuclear genes as precursors and are targeted to the chloroplast by a CTP that is removed during the import steps. Examples of chloroplast proteins include the small subunit (RbcS2) of ribulose-1,5,-bisphosphate carboxylase, ferredoxin, ferredoxin oxidoreductase, the light-harvesting complex protein I and protein II, and thioredoxin F. It has been demonstrated in vivo and in vitro that non-chloroplast proteins may be targeted to the chloroplast by use of protein fusions with a CTP and that a CTP is sufficient to target a protein to the chloroplast. Incorporation of a suitable chloroplast transit peptide, such as, the *Arabidopsis thaliana* EPSPS CTP (Klee *et al.,* 1987), and the *Petunia hybrida* EPSPS CTP (della-Cioppa *et al.,* 1986) has been shown to target heterologous protein to chloroplasts in transgenic plants. The *Zea mays* DapA CTP (SEQ ID NO:6, herein referred to as TS-Zm.DapA) of the present invention has utility to provide direction of the linked DHDPS polypeptide into the plastids of the seed. Those skilled in the art will recognize that various chimeric constructs can be made that utilize the functionality of a particular CTP to import a heterologous DHDPS polypeptide into the plant cell plastid. For a description of the use of a chloroplast transit peptide see U.S. Patent 5,188,642.

Table 1 illustrates combinations of promoters and other regulatory elements operably linked to LKR gene suppression molecules and DHDPS coding molecules and their resulting effect on lysine levels in corn plants and kernels. One skilled in the art would know that exogenous DNA molecules having similar function can be substituted with those illustrated in Table 1 and tested in the methods described herein or related methods that are useful for the evaluation of lysine levels in plant tissues, kernels or processed products.

DNA constructs of the present invention may optionally contain a selectable or scorable marker molecule. Of particular importance are selectable marker molecules that also provide a valuable agronomic trait, for example, herbicide tolerance. Herbicides for which transgenic plant tolerance has been demonstrated and the method of the present invention can be applied, include but are not limited to: glyphosate, glufosinate, sulfonylureas, imidazolinones, bromoxynil, dalapon, cyclohexanedione, protoporphyrinogen oxidase inhibitors, and isoxaflutole herbicides. Polynucleotide molecules encoding proteins involved in herbicide tolerance are known in the art, and include a polynucleotide molecule encoding 5-enolpyruvylshikimate-3-phosphate synthase (EPSPS, described in U.S. Patent Nos. 5,627,061; 5,633,435; 6,040,497; Padgette *et al.,* 1996; and Penaloza-Vazquez *et al.,* 1995; and *aroA* (U.S. Patent No. 5,094,945) for glyphosate tolerance; bromoxynil nitrilase (*Bxn*) for Bromoxynil tolerance (U.S. Patent No. 4,810,648); phytoene desaturase (*crtI,* Misawa *et al.,* 1993 and 1994); for tolerance to norflurazon, acetohydroxyacid synthase (AHAS, *aka* ALS, Sathasiivan *et al.,* 1990); and the *bar* gene for tolerance to glufosinate and bialaphos (DeBlock *et al.,* 1987).

Herbicide tolerance is a desirable phenotype for crop plants. DNA constructs of the present invention preferably contain an expression cassette that provides glyphosate tolerance in the transformed corn plant. The expression cassette additionally allows the selection of transformed corn cells on a media containing glyphosate. The expression cassette preferably contains a promoter that directs the transcription of a gene coding for a glyphosate resistant EPSPS in all of the cells of the transformed plant. In particular, the glyphosate tolerant expression cassette of the present invention comprises the rice actin 1 promoter and intron (P-Os.Act1, I-Os.Act1, U.S. Patent 5,641,876) operably linked to a chloroplast transit peptide-encoding polynucleotide and a polynucleotide encoding a glyphosate resistant EPSPS isolated from *Agrobacterium* strain CP4 (herein referred to as aroA:CP4 EPSPS, U.S. Patent 5,633,435). N-phosphonomethylglycine, also known as glyphosate, is a well known herbicide that has activity on a broad spectrum of plant species. Glyphosate is the active ingredient of Roundup® herbicide (Monsanto Co., St. Louis, MO), a safe herbicide having a desirably short half life in the environment. When applied to a plant surface, glyphosate moves systemically through the plant. Glyphosate is toxic to plants by blocking the shikimic acid pathway which provides a precursor for the synthesis of aromatic amino acids. Specifically, glyphosate affects the conversion of phosphoenolpyruvate and 3-phosphoshikimic acid to 5-enolpyruvylshikimate 3-phosphate by inhibiting the enzyme 5-enolpyruvylshikimate 3-phosphate synthase (hereinafter referred to as EPSP synthase or EPSPS). For purposes of the present invention, the term "glyphosate" should be considered to include any herbicidally effective form of N-phosphonomethylglycine (including any salt thereof) and other forms which result in the production of the glyphosate anion *in planta.* Glyphosate as N-phosphonomethylglycine and its salts are used as components of synthetic culture media for the selection of bacterial and plant tolerance to glyphosate or used to determine enzyme resistance in *in vitro* biochemical assays. Examples of commercial formulations of glyphosate include, without restriction, those sold by Monsanto Company under the trademarks ROUNDUP®, ROUNDUP® ULTRA, ROUNDUP® ULTRAMAX, ROUNDUP® CT, ROUNDUP® EXTRA, ROUNDUP® BIACTIVE, ROUNDUP® BIOFORCE, RODEO®, POLARIS®, SPARK® and ACCORD® herbicides, all of which contain glyphosate as its isopropylammonium salt; those sold by Monsanto Company as ROUNDUP® DRY and RIVAL® herbicides, which contain glyphosate as its ammonium salt; that sold by Monsanto Company as ROUNDUP® GEOFORCE, which contains glyphosate as its sodium salt; that sold by Monsanto Company as ROUNDUP® WEATHERMAX, which contains glyphosate as its potassium salt; and that sold by Zeneca Limited as TOUCHDOWN® herbicide, which contains glyphosate as its trimethylsulfonium salt. Glyphosate herbicide formulations can be safely used over the top of glyphosate tolerant crops to control weeds in a field at rates as low as 0.6 kg/ha (8 ounces/acre) and up to 4.5 kg/ha (64 ounces/acre). Experimentally, glyphosate has been applied to glyphosate tolerant crops at rates as low as 0.3 kg/ha (4 ounces/acre) and up to or exceeding 9.0 kg/ha (128 ounces/acre) with no substantial damage to the crop plants. The selection of application rates for a glyphosate formulation that constitute a biologically effective dose is within the skill of the ordinary agricultural technician. To illustrate that production of transgenic plants with herbicide resistance is a capability of those of ordinary skill in the art, reference is made to U.S. Patent Application Publications 2003/0106096A1 and 2002/0112260A1 and U.S. Patents 5,034,322; 6,107,549 and 6,376,754. The present invention provides a corn plant with increased lysine and is optionally glyphosate tolerant.

During transformation, the exogenous DNA may be introduced randomly, i.e. at a non-specific location, in the plant genome. In some cases, it may be useful to target an exogenous DNA insertion in order to achieve site-specific integration, for example, to replace an existing gene sequence or region in the genome. In some other cases it may be useful to target an exogenous DNA integration into the genome at a predetermined site from which it is known that gene expression occurs. Several site-specific recombination systems exist that are known to function in plants include Cre/lox as disclosed in U.S. Patent 4,959,317 and FLP/FRT as disclosed in U.S. Patent 5,527,695.

Useful selectable marker genes include those conferring resistance to antibiotics such as ampicillin, kanamycin (nptII), hygromycin B (*aph IV*) and gentamycin (*aac3* and *aacC4*), or selectable marker genes as described in U.S. Patents 5,550,318; 5,633,435; 5,780,708 and 6,118,047. Screenable markers that provide an ability to visually identify transformants can also be employed, for example, a gene expressing a colored or fluorescent protein such as a luciferase or green fluorescent protein (GFP) or a gene expressing a beta-glucuronidase or *uid*A gene (GUS) for which various chromogenic substrates are known.

### Gene suppression

DNA constructs of the present invention provide expression of an anti-sense RNA gene suppression molecule specific to the endogenous LKR gene (lysine-ketoglutarate reductase (LKR, a bifunctional enzyme in plants, also known as saccharopine dehydrogenase, SDH) in a transgenic plant cell. Such a DNA construct comprises a promoter active in the tissue targeted for enzyme suppression, and a transcribable DNA element having a sequence that is complementary to the polynucleotide sequence of an LKR gene targeted for suppression. The targeted gene element copied for use in transcribable DNA in the gene suppression construct can be a promoter element, an intron element, an exon element, a 5' UTR element, or a 3'UTR element. Although the minimum size of DNA copied from sequence of a gene targeted for suppression is believed to be about 21 or 23 nucleotides; larger nucleotide segments are preferred, for example up to the full length of a targeted gene. The DNA element can comprise multiple parts of a gene, for example nucleotides that are complementary to contiguous or separated gene elements of UTR, exon and intron. Such constructs may also comprise other regulatory elements, DNA encoding transit peptides, signal peptides, selective markers and screenable markers as desired. To form an anti-sense-oriented RNA loop the complementary DNA element is conveniently not more than about one-half the length of the anti-sense-oriented DNA element, often not more than one-third the length of said anti-sense-oriented DNA element, for example not more than one-quarter the length of said anti-sense-oriented DNA element. The overall lengths of the combined DNA elements can vary. For instance, the anti-sense-oriented DNA element can consist of from 500 to 5000 nucleotides and the complementary DNA element can consist of from 50 to 500 nucleotides.

The anti-sense transcription unit can be designed to suppress multiple genes or gene family members where the DNA is arranged with two or more anti-sense-oriented elements from different genes targeted for suppression followed by a complementary sense-oriented element, for example complementary to at least a part of the 5'most anti-sense element.

Gene suppression includes any of the well-known methods for suppressing transcription of a gene or the accumulation of the mRNA corresponding to that gene thereby preventing translation of the transcript into protein. Posttranscriptional gene suppression is mediated by transcription of integrated recombinant DNA to form double-stranded RNA (dsRNA) having homology to a gene targeted for suppression. This formation of dsRNA most commonly results from transcription of an integrated inverted repeat of the target gene, and is a common feature of gene suppression methods known as anti-sense suppression, co-suppression and RNA interference (RNAi). Transcriptional suppression can be mediated by a transcribed dsRNA having homology to a promoter DNA sequence to effect what is called promoter *trans* suppression.

More particularly, posttranscriptional gene suppression by inserting a recombinant DNA construct with anti-sense oriented DNA to regulate gene expression in plant cells is disclosed in U.S. Patent 5,107,065 (Shewmaker, et al.) and U.S. Patent 5,759,829 (Shewmaker, et al.). Transgenic plants transformed using such anti-sense oriented DNA constructs for gene suppression can comprise integrated DNA arranged as an inverted repeat that results from insertion of the DNA construct into plants by *Agrobacterium-mediated* transformation, as disclosed by Redenbaugh *et al.* (1992). Inverted repeat insertions can comprise a part or all of the T-DNA construct, for example, an inverted repeat of a complete transcription unit or an inverted repeat of a transcription terminator sequence. Screening for inserted DNA comprising inverted repeat elements can improve the efficiency of identifying transformation events effective for gene silencing whether the transformation construct is a simple anti-sense DNA construct which must be inserted in multiple copies or a complex inverted repeat DNA construct (for example an RNAi construct) which can be inserted as a single copy. A DNA construct of the present invention can contain an inverted repeat inserted within a regulatory element, for example, an intron. The inverted repeat comprising a DNA molecule that is homologous and complementary to an endogenous LKR gene of the host plant cell. In the present invention, a DNA molecule (SEQ ID NO:9, dsSDH bfx, also referred to as LKR dsRNA bfx) is homologous to a portion of the DNA coding for a *Zea mays* LKR/SDH (SEQ ID NO:2), and is utilized to suppress expression of the native corn LKR/SDH genes. The bfx annotation refers to a dsRNA molecule sequence used for gene suppression that was specifically selected by an analysis of the target molecule and exclusion of related target sequences found in some other organisms. In some constructs described herein, the LKR dsRNA bfx fragment is embedded in an intron (I-Zm.DnaK embedded Zm.LKR dsRNA bfx, SEQ ID NO:10). Additional polynucleotides homologous and complementary to the *Zea mays* LKR gene sequence can be selected for use in the present invention.

Suppression achieved by insertion mutations created by transposable elements may also prevent gene function. For example, in many dicot plants, transformation with the T-DNA of *Agrobacterium* may be readily achieved and large numbers of transformed plants can be rapidly obtained. Also, some species such as *Zea mays* have lines with active transposable elements that can efficiently be used for the generation of large numbers of insertion mutations, while some other species lack such options. Mutant plants produced by *Agrobacterium* or transposon mutagenesis and having altered expression of a polypeptide of interest can be identified using the polynucleotides of the present invention. For example, a large population of mutated plants may be screened with polynucleotides encoding the polypeptide of interest to detect mutated plants having an insertion in the gene encoding the polypeptide of interest.

### Protein Molecules

Proteins of the present invention that represent whole proteins or at least a sufficient portion of the entire protein to impart the relevant biological activity of the protein, for example increased lysine content in a transgenic maize kernel. The term "protein" also includes molecules consisting of one or more polypeptide chains. Thus, a protein useful in the present invention may constitute an entire gene product or one or more functional portions of a natural protein that provides the agronomic trait of this invention, i.e. increased lysine.

Homologs of the proteins of the present invention may be identified by comparison of the amino acid sequence of the DHDPS (SEQ ID NO:7), or aspartate kinase (SEQ ID NO:8) proteins to amino acid sequences of proteins from the same or different plant or bacterial sources, for example manually or by using known homology-based search algorithms such as those commonly known and referred to as BLAST, FASTA, and Smith-Waterman.

A further aspect of the invention provides coding sequences which encode functional homologous proteins which differ in one or more amino acids from those of the DHDPS or aspartate kinase proteins provided herein as the result of one or more of the well-known conservative amino acid substitutions, for example valine is a conservative substitute for alanine and threonine is a conservative substitute for serine. When such a homologous protein is expressed in a transgenic plant, the homologous protein will affect the transgenic plant in a substantially equivalent manner as the DHDPS or aspartate kinase proteins.

Conservative substitutions for an amino acid within the native protein sequence can be selected from other members of a class to which the naturally occurring amino acid belongs. Representative amino acids within these various classes include, but are not limited to: (1) acidic (negatively charged) amino acids such as aspartic acid and glutamic acid; (2) basic (positively charged) amino acids such as arginine, histidine, and lysine; (3) neutral polar amino acids such as glycine, serine, threonine, cysteine, tyrosine, asparagine, and glutamine; and (4) neutral nonpolar (hydrophobic) amino acids such as alanine, leucine, isoleucine, valine, proline, phenylalanine, tryptophan, and methionine. Conserved substitutes for an amino acid within a native amino acid sequence can be selected from other members of the group to which the naturally occurring amino acid belongs. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine. Naturally conservative amino acids substitution groups are: valine-leucine, valine-isoleucine, phenylalanine-tyrosine, lysine-arginine, alanine-valine, aspartic acid-glutamic acid, and asparagine-glutamine.

A further aspect of the invention comprises proteins which differ in one or more amino acids from those of a described DHDPS or aspartate kinase proteins sequences as the result of deletion or insertion of one or more amino acids in a native sequence. When such a homologous protein is expressed in a transgenic plant, the homologous protein will affect the transgenic plant in a substantially equivalent manner, for example, result in increased lysine content in the maize kernel.

Proteins of the present invention that are variants of the proteins provided herein will generally demonstrate significant identity with the proteins provided herein, such as at least 50% or more, for example, at least 60% or 70% identity with DHDPS or aspartate kinase proteins. Useful proteins also include those with higher percentage identity with the amino acids in a protein segment of DHDPS or aspartate kinase proteins, for example, 80%, 90%, 95%, 98% or up to 99% identity.

### Transformation Methods and Plants

As used herein, the term "maize" means *Zea mays,* also known as corn, and includes all plant varieties that can be bred with maize, including wild maize species.

Methods and compositions for transforming plants by introducing an exogenous DNA into a plant genome in the practice of this invention can include any of the well-known and demonstrated methods. Preferred methods of plant transformation are microprojectile bombardment as illustrated in U.S. Patents 5,015,580; 5,550,318; 5,538,880; 6,160,208; 6,399,861 and 6,403,865 and Agrobacterium-mediated transformation as illustrated in U.S. Patents 5,635,055; 5,824,877; 5,591,616; 5,981,840 and 6,384,301 and U.S. Patent Publication 20030196219. Microparticle-mediated transformation refers to the delivery of DNA coated onto microparticles that are propelled into target tissues by several methods. Agrobacterium-mediated transformation is achieved through the use of a genetically engineered soil bacterium belonging to the genus *Agrobacterium.* Several *Agrobacterium* species mediate the transfer of a specific DNA plasmid known as "T-DNA," which can be genetically engineered to carry any desired piece of DNA into many plant species.

As used herein a "transgenic" organism is one whose genome comprises exogenous DNA or isolated DNA. The transgenic organism may be a plant, animal, insect, fungus, bacterium, or virus. As used herein "transgenic plant" means a stably transformed plant or a progeny plant of any subsequent generation descended therefrom, wherein the DNA of the plant or progeny thereof contains exogenous DNA. The transgenic plant may additionally contain sequences that are native to the plant being transformed, but wherein the exogenous DNA has been altered in order to alter the level or pattern of expression of the gene.

As used herein, a "stably" transformed plant is a plant in which the exogenous DNA is heritable. The exogenous DNA may be heritable as a fragment of DNA maintained in the plant cell and not inserted into the host genome. Preferably, the stably transformed plant comprises the exogenous DNA inserted into the chromosomal DNA in the nucleus, mitochondria, or chloroplast, most preferably in the nuclear chromosomal DNA.

As used herein an "Rₒ transgenic plant" is a plant that has been directly transformed with an exogenous DNA or has been regenerated from a cell, callus or cell cluster that has been transformed with an exogenous DNA. As used herein "progeny" means any subsequent generation, including the seeds and plants therefrom, which is derived from a particular parental plant or set of parental plants; the resultant progeny may be highly hybridized or substantially homozygous, depending upon pedigree. Progeny of a transgenic plant of this present invention can be, for example, self-pollinated, crossed to another transgenic plant, crossed to a nontransgenic plant, and/or back crossed to an ancestor.

The seeds of the plants of this invention can be harvested from fertile transgenic plants and can be used to grow progeny generations of plants of this invention, including a hybrid plant line comprising in its genome an exogenous DNA construct as listed in Table 1 of the present invention, which provides the benefit of increased lysine in the maize kernel and optionally tolerance to glyphosate herbicide.

A transgenic "event" is produced by transformation of a plant cell with an exogenous DNA construct, the regeneration of a plant resulting from the specific insertion of the exogenous DNA into the genome of the plant, and selection of a particular plant characterized by event DNA. Each event is therefore a unique individual that is distinguishable from other events by the specific location of the genomic insertion of the exogenous DNA construct of the present invention. Detection of the event DNA is performed by any number of DNA detection methods known in the art and can be conducted on plants tissues, seeds, and processed products of the event that contain DNA. Typically, a number of plant cells are transformed, each potentially representing a different integration event, producing a population of plants from which a particular plant is selected. Since the locus of integration is generally stable across repeated cycles of plant reproduction, the term "event" refers to the original Rₒ transformant and progeny of the transformant that include the exogenous DNA inserted into a particular and unique location in the genome, *i.e.*, event DNA. Progeny produced by a sexual outcross, a self-cross, or repeated backcrossing, wherein at least one of the plants used in the breeding are descendants at any generation from the original Rₒ transformant, contain the same event DNA.

In processing of the seeds and grain of this invention to produce feed and food products, the normal plant tissue structures will be destroyed, the plant cells may be ruptured and the native DNA and the exogenous plant expression cassettes may not remain entirely intact, particularly when high temperatures, extraction solvents and the like are used. Nevertheless the unique processed products enabled by the practice of this invention can readily be identified as comprising fragments of the native DNA and fragments of the exogenous plant expression cassettes. By "fragments" is meant isolatable or amplifiable DNA segments of at least 20 base pairs in length. Such fragments can be sequenced or restriction mapped to confirm sequence identity to the native DNA or the exogenous expression cassettes of the starting material.

### EXAMPLES

The following examples are included to demonstrate examples of certain preferred embodiments of the present invention. It should be appreciated by those of skill in the art that the techniques disclosed in the examples that follow represent approaches the inventors have found function well in the practice of the present invention, and thus can be considered to constitute examples of preferred modes for its practice.

### Example 1

### DNA constructs and plant transformation

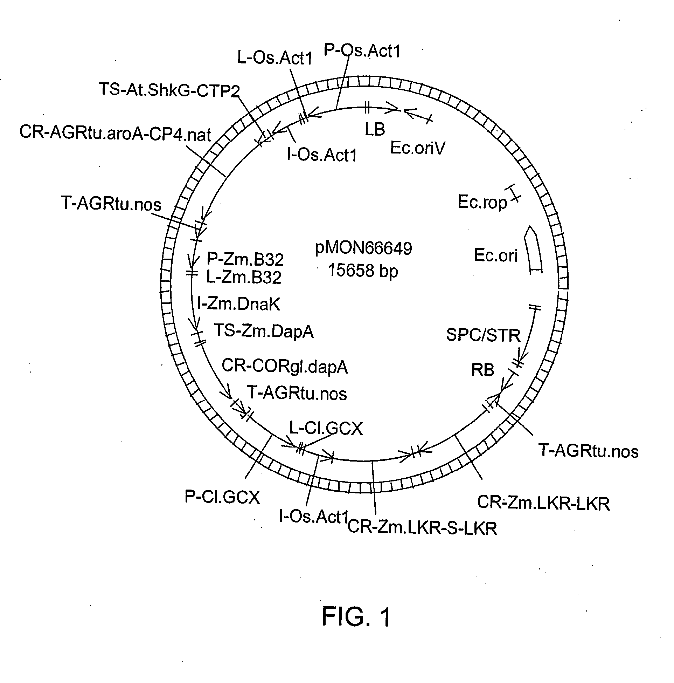

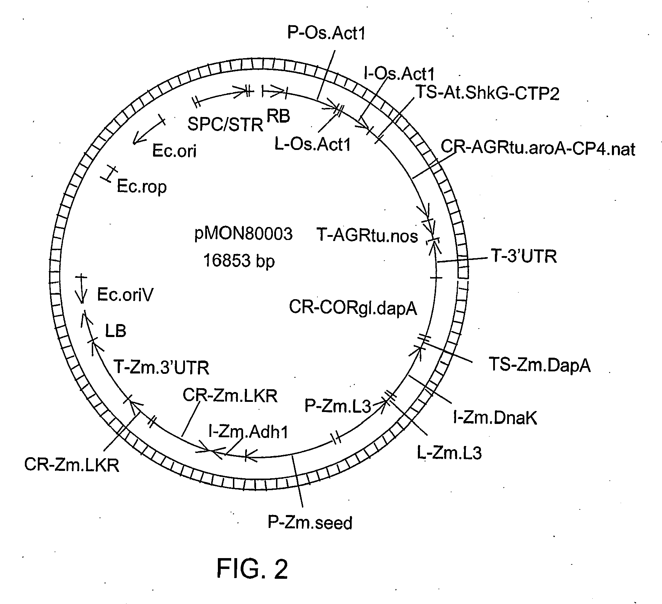

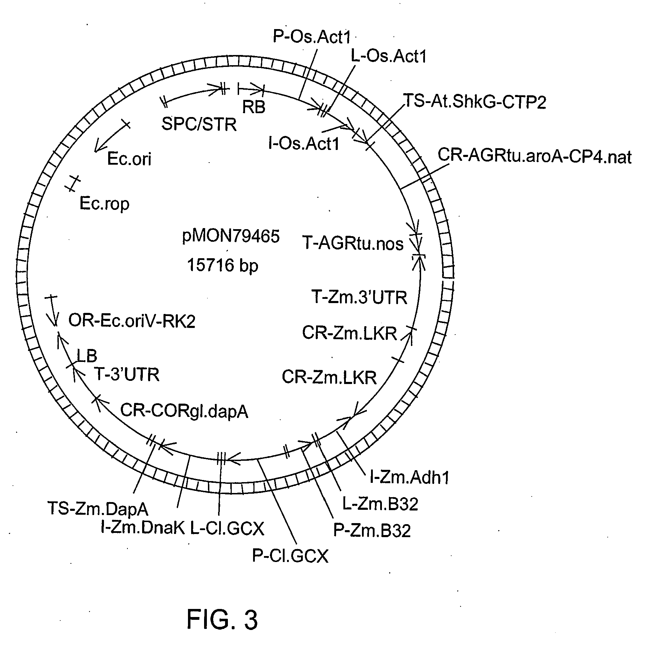

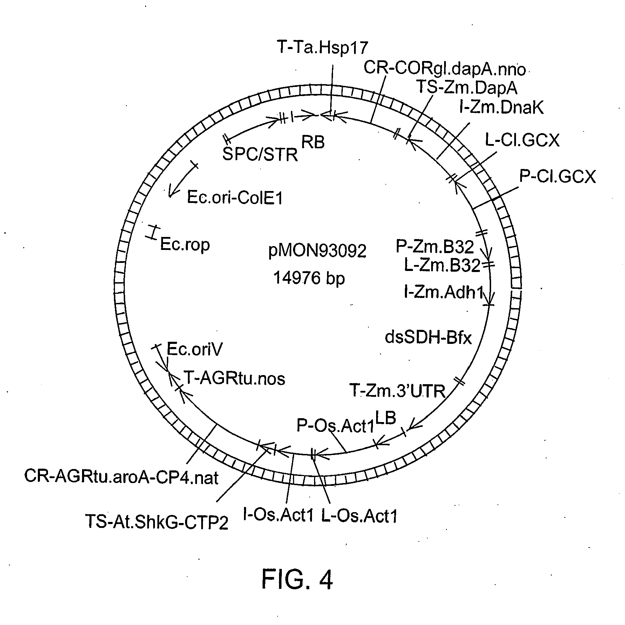

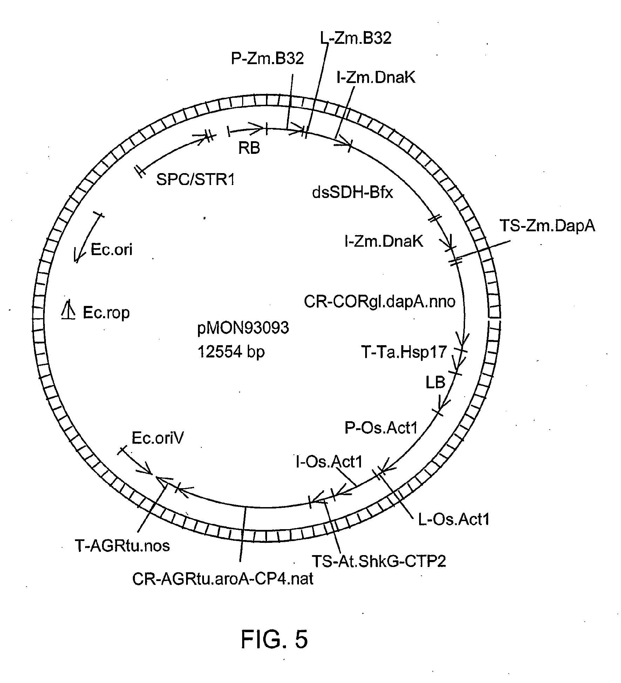

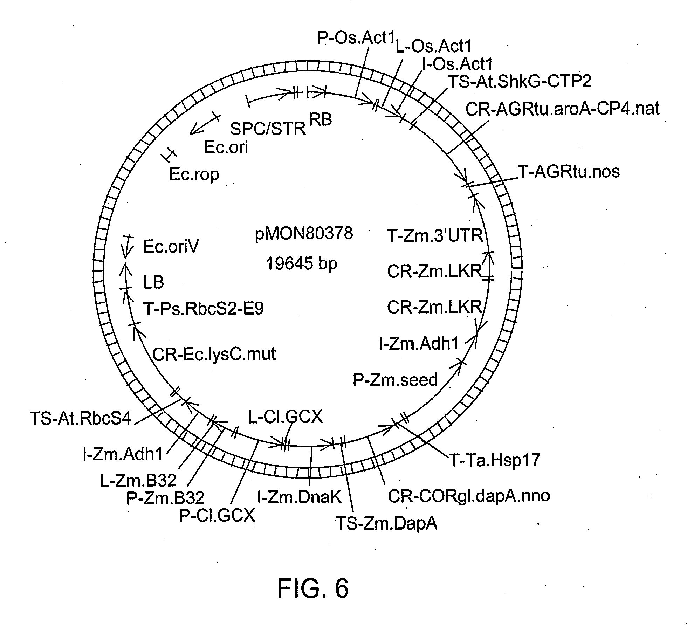

DNA constructs of the present invention comprise regulatory molecules operably linked to the DNA of interest. In particular, a DNA construct comprises a promoter, an intron, a 5' leader, a coding or noncoding molecule and a 3' untranslated region. The DNA constructs of the present invention were prepared using various molecular methods and tools, for example, those described in Sambrook *et al.,* and variations of these methods that are known by those skilled in the art of DNA manipulation can be conducted to produce substantially similar constructs. Methods such as, PCR amplification, cloning and subcloning methods were used to operably link promoters, leaders, introns, transit peptides, coding molecules, noncoding molecules, and 3' untranslated regions as listed in Table 1 in operable configurations and further illustrated in Figures 1-6. The DNA constructs were cloned into one or more plasmid backbones appropriate for use in *Agrobacterium-mediated* transformation of maize cells, although other plasmid backbones can be selected, for example, high copy number plasmids to isolate DNA fragments for particle gun mediated transformation, or plasmids that are compatible with other bacterial hosts. Thirty-five constructs listed in Table 1 were made to test combinations of expression strategies for expression of the lysine feedback resistant DHDPS polypeptide (SEQ ID NO:7) and repression of lysine catabolism by gene suppression of the maize endogenous LKR/SDH gene (SEQ ID NO:2) or expression of an aspartate kinase (SEQ ID NO:8). For example, these strategies included: 1) expression of LKR-dsRNA in the embryo; 2) expression of both the DHDPS polypeptide and the LKR-dsRNA in the embryo; 3) expression of both the DHDPS polypeptide and the LKR-dsRNA in the endosperm; 4) expression of the DHDPS polypeptide in the endosperm and LKR-dsRNA in the embryo; 5) expression of the DHDPS polypeptide and aspartate kinase and expression of the LKR-dsRNA.

### Preparation of transgenic plants

Corn tissues (LH198 or LH244 inbred lines) that comprise immature embryos or callus are isolated for transformation, and the DNA constructs of the present invention are inserted into the corn genome using *Agrobacterium* mediated transformation methods (for example, U.S. Patents 5,635,055; 5,824,877; 5,591,616; 5,981,840 and 6,384,301). *Agrobacterium* transformed to comprise the DNA constructs listed in Table 1 are inoculated onto the corn tissue. Following exposure to the transformed bacteria, standard transformation protocols are used to propagate and select for transformed corn cells using media supplemented with glyphosate herbicide. Following selection of cells surviving exposure to the glyphosate herbicide, Rₒ transgenic plants were regenerated to maturity. Seeds are collected from the fertile transgenic plants following either self-crossing or out-crossing to a second corn plant. Molecular analyses are carried out to characterize the transgenic insertions. PCR, Southern blotting, Taqman® assay or other methodologies known to those of skill in the art of molecular biology are used to identify events comprising single, intact insertions of the DNA constructs or zygosity analysis. The progeny seed is planted and the progeny plants grown for lysine analysis of the tissues and progeny seed.

### Example 2

### Determination of lysine content of the maize tissues and seed

Various methods are useful to determine the lysine content of plant tissues, seeds and processed products, for example, in the present invention liquid chromatography-mass spectrophotometry/mass spectrophotometry (LC-MS-MS) was used to analyze free lysine (parts per million, ppm) in the corn kernels of the various events identified as transformed with a DNA construct as listed in Table 1 and the results of this analysis are described in Table 1 and Table 2. Individual mature corn kernel samples of each event were first weighed, ground to a fine, homogeneous powder and extracted with an extraction solvent comprising methanol, water, and formic acid. In situations where kernels were bulked, approximately 30 mg of ground powder was used. Both liquid chromatography and multiple-reaction-monitoring (MRM) mass spectrometric techniques were used to separate lysine in the sample extract. After the separation, lysine was quantified using its mass spectrometry peak area against its corresponding standard calibration curve which was prepared using a deuterated d₄-lysine internal standard (IS).

In another method, lysine content of corn kernels was based upon evaluation of the free amino acids by high performance liquid chromatography (HPLC). Individual kernels or pools of kernels of each event were ground to a fine, homogenous powder as described, and in this instance, approximately 30 mg of powder was used for analysis. Amino acids were extracted with 5% trichloroacetic acid and amino acid detection was achieved through a pre-column primary amine derivatization with o-phthalaldehyde (OPA). Using reverse-phase chromatography, separation is achieved through the hydrophobicity of the R-groups located on each amino acid. To help stabilize the fluorophor, a thiol is added such as 2-mercaptoethanol (SHCH₂CH₂OH) or 3-mercaptopropionic acid (SHCH₂CH₂COOH).

Free lysine levels (ppm) measured in the transgenic events produced by transformation with each of the constructs were averaged and summarized in Table 1. The generation of the seed (F1, F2, F3 and F4) assayed is also identified. Free lysine level measurements on R1 seed of transgenic corn events containing pMON80378 is presented in Table 2. Lysine levels were surprisingly high in 5 of the 6 events assayed. Lysine levels can be further adjusted by breeding corn events that contain various combinations of the plant expression cassettes described in table 1 such that the hybrid seed of the cross of parents containing one or more of the expression cassettes have the desired level of lysine. An inbred parents that, for example, contains the CordapA and *E.coli lysC* (wild type or Ec.lysC.mut, SEQ ID NO:11, a DNA molecule that encodes for a protein modified at four positions, S2A, C58G, T352I and A401G) expression cassettes can be bred with a parent with the LKR dsRNA expression cassette to yield a hybrid progeny seed with lysine levels that are more than additive of the lysine levels of the seed of either parent. Other combinations, such as, a CordapA and the LKR dsRNA containing parent can be crossed with a LysC parent, or a LysC and LKR dsRNA parent can be crossed with a CordapA parent to provide unexpected high levels of lysine in the hybrid seed.

**Table 1. Lysine (ppm) average per generation assayed from greenhouses and various field tests.**

| **Genotype** | **F1** | **F2 (R1)** | **F3 (R2)** | **F4 (R3)** |
|---|---|---|---|---|
| 1. pMON65178: P-Zm.seed/I-Os.Actl/TS-Zm.DapA-CORgl.dapA /T-3'UTR | | | | |
| LH198/HiII | - | 1720 ppm | 1825 ppm | 2133 ppm |
| 2. pMON66568: P-Cl.Gcx/I-Os.Act1/Zm.LKR antisense /T-3'UTR | | | | |
| LH198/HiII | - | 31 ppm | 56 ppm | - |
| 3. pMON66646: P-Cl.Gcx/I-Os.Act1/Zm.LKR-dsRNA/T-3'UTR | | | | |
| LH198/HiII | | 1279 ppm | 1893 ppm | 2575 ppm |
| 4. pMON66649: P-Zm.B32/I-Zm.DnaK/TS-Zm.DapA-CORgl.dapA/T-3'UTR > <P-Cl.Gcx/ I-Os.Actl/Zm.LKR-dsRNA/T-3'UTR | | | | |
| LH198/HiII | - | 1871 ppm | 4556 ppm | - |
| 5. pMON79454: P-Zm.L3/ I-Zm.DnaK / TS-Zm.Dap pA-CORgl.dapA /3 'UTR | | | | |
| LH244 | 862 ppm | 1274 ppm | 3084 ppm | - |
| 6. pMON80002: P-Zm.seed/I-Zm.Adh1/Zm.LKR-dsRNA/T-3'UTR | | | | |
| LH244 | 38 ppm | 147 ppm | 330ppm | - |
| 7. pMON80003: P-Zm.L3/ I-Zm.DnaK /TS-Zm.DapA-CORgl.dapA /T-3'UTR> < P-Zm.seed/I-Zm.Adh1/Zm.LKR dsRNA/T-3'UTR | | | | |
| LH244 | 1660 ppm | 2223ppm | 4213 ppm | - |
| 8. pMON80000: P-L3/ I-Zm.DnaK /TS-Zm.DapA-CORgl.dapA /T-3'UTR | | | | |
| LH244 | - | 1157 ppm | 2560 ppm | - |
| 9. pMON80001: P-Cl.Gcx/ I-Zm.DnaK /TS-Zm.DapA-CORgl.dapA/T-3'UTR | | | | |
| LH244 | ppm | 44 ppm | - | - |
| 10. PMON80354: P-Cl.Gcx/I-Zm.DnaK / TS-Zm.DapA-CORgl.dapA.nno / T-3'UTR | | | | |
| LH244 27 | ppm | 47 ppm | - | - |
| 11. PMON80355: P-Zm.L3/ I-Zm.DnaK/TS-Zm.DapA-CORgl.dapA.nno /T- 3'UTR | | | | |
| LH244 930 | ppm | 2065 ppm | - | - |
| 12. PMON79901: P-Zm.Em/I-Zm.Adh1/Ec.lysC/T-Ps.RbcS2:E9> <P-Zm.L3/I-Zm.DnaK/TS-Zm.DapA-CORgl.dapA /T-3'UTR | | | | |
| LH244 1 | 385 ppm | 3685 ppm | - | - |
| 13. PMON79900: P-Zm.L3/ I-Zm.DnaK/TS-Zm.DapA-CORgl.dapA /3'UTR >< P-Zm.Em/I-Zm.Adh1/Ec.lysc/3'UTR | | | | |
| LH244 2 | 150 ppm | 3688 ppm | - | - |

| 14. PMON79464: | P-Zm.B32/I-Zm.Adh1/Zm.LKR | | dsRNA/T-3'UTR | |
|---|---|---|---|---|
| LH244 | 159 ppm | 720 ppm | 567 ppm | - |
| 15. PMON79465: P-Zm.B32/I-Zm.Adhl/Zm.LKR dsRNA intermal fragment/T-3'UTR> | | | | |
| <P-Cl.Gcx/ | I-Zm.DnaK | /TS-Zm.DapA-CORgl.dapA/T-3'UTR | | |
| LH244 | 1470 ppm | 3171 ppm | 4158 ppm | - |
| 16. PMON74130: P-Cl.Gcx/I-Os.Act1/LKR-dsRNA/T-3'UTR> <PZm.B32/ I-Zm.DnaK / TS-Zm.DapA-CORgl.dapA.nno/T-3'UTR | | | | |
| LH244 | - | 3443 ppm | | |
| 17. pMON80371: P-Zm.B32/I-Zm.Adhl/Ec.lysC/T-3'UTR><P-Zm.Gcx/ I-Zm.DnaK / TS-Zm.DapA-CORgl.dapA.nno /T-3'UTR | | | | |
| LH244 | - | - | | |
| 18. pMON80374:P-Zm.B32/ I-Zm.Adh1/Ec.lysC/3'UTR><P-Cl.Gcx/ I-Zm.DnaK/ TS-Zm.DapA-CORgl.dapA.nno /T-Ta.hsp17 | | | | |
| LH244 | - | - | | |
| 19. pMON80378: P-Zm.B32/ I-Zm.Adhl /Ec.lysC/3'UTR ><P-Zm.Gcx/ I-Zm.DnaK/ TS-Zm.DapA-CORgl.dapA.nno /3'UTR > <P-Zm.seed/Zm.LKR dsRNA intermal fragment /T-3'UTR | | | | |
| LH244 | - | 8974 ppm | - | |
| 20. pMON93072: P-Zm.B32/ I-Zm.DnaK embedded Zm.LKR dsRNA intermal fragment /TS-Zm.DapA-CORgl.dapA.nno /3'UTR><P-Zm.Gcx/I-Zm.Adh1/Ec.lysC/T-3'UTR | | | | |
| LH244 | | | | |
| 21. pMON93073:P-Cl.Gcx/I-Zm.DnaK/ TS-Zm.DapA-CORgl.dapA.nno /3'UTR><P-Zm.B32/ I-Zm.Adhl / Zm.LKR dsRNA intermal fragment / T-3'UTR | | | | |
| LH244 | - | - | | |
| 22. pMON93066: P-B32/ I-Zm.DnaK embedded Zm.LKR dsRNA intermal fragment/ TS-Zm.DapA-CORgl.dapA.nno)/ T-3'UTR | | | | |
| LH244 | - | 3648 ppm | - | |
| 23. pMON93067: P-B32/ I-Zm.DnaK embedded Zm.LKR dsRNA intermal fragment / TS-Zm.DapA-CORgl.dapA.nno /T-3'UTR | | | | |
| LH244 | | | | |
| 24. pMON93063: P-Zm.B32/I-Zm.Adhl / Zm.LKR dsRNA intermal fragment /T-3'UTR > <P-Cl.Gcx/I-Zm.DnaK/ TS-Zm.DapA-CORgl.dapA.nno/T-3'UTR | | | | |
| LH244 | | | | |
| 25. pMON93064: P-Zm.Em/ I-Zm.Adhl /Ec.lysC/3'UTR > <P-Zm.L3/ I-Zm.DnaK/ TS-Zm.DapA-CORgl.dapA.nno)/ T-3'UTR | | | | |
| LH244 | | | | |
| 26. pMON80370: P-Zm.L3/ I-Zm.DnaK embedded Zm.LKR dsRNA intermal fragment /TS-Zm.DapA-CORgl.dapA.nno /T-3'UTR | | | | |
| LH244 | - | 645 ppm | - | |
| 27. pMON79469: P-Zm.B32/ I-Zm.Adhl /Zm.LKR-dsRNA/ 3'UTR><P-Cl.Gcx/I-Zm.DnaK/ TS-Zm.DapA-Zm.DHDPS/ T-3'UTR | | | | |
| LH244 | - | 182 ppm | - | - |
| 28. pMON79470: P-Zm.B32/ I-Zm.Adhl /Zm.LKR internal fragment/3'UTR><P-Cl.Gcx/I-Zm.DnaK /TS-Zm.DapA-DHDPS/T-3'UTR | | | | |
| LH244 | - | 368 ppm | - | - |
| 29. pMON93065:P-Zm.seed/ I-Zm.Adh1 /Zm.LKR internal fragment / 3'UTR > <P-Zm.L3/ I-Zm.DnaK/TS-Zm.DapA-CORgl.dapA.nno/T-3'UTR | | | | |
| LH244 | | | | |
| 30. pMON93092: P-Cl.Gcx/I-Zm.DnaK/TS-Zm.DapA-CORgl.dapA.nno/T-Ta.Hap17><P-Zm.B32/I-Zm.Adhl/Zm.LKR dsRNA bfx/T-3'UTR | | | | |
| LH244 | | | 4478 ppm | |
| 31. pMON93093: P-Zm.B32/I-Zm.DnaK-LKR dsRNA bfx -I-Zm.DnaK/TS-Zm.DapA-CORgl.dapA.nno/T-Ta.Hap 17 | | | | |
| LH244 | | | 3041 ppm | |
| 32. pMON93083: P-Cl.Gcx/I-Zm.DnaK/TS-Zm.DapA- CORgl.dapA.nno /T-Ta.Hsp17, P-Zm.B32/1-Zm.Adhl/Zm.LKR dsRNA bfx/T-3'UTR> | | | | |
| LH244 | | | | |
| 33. pMON93084: P-Cl.Gcx/I-Zm.DnaK/ TS-Zm.DapA-CORgl.dapA.nno/T-Ta.Hsp17, P-Zm.B32/I-Zm.Adh1/Zm.LKR dsRNA bfx)/T-3'UTR> | | | | |
| LH244 | | | | |
| 34. pMON80380: RB <P-Cl.Gcx/I-Zm.Adh1/Ec.lysC/T-Ps.RbcS2:E9><P- Zm. B32/I-Zm.DnaK-Zm.LKR dsRNA-Zm.DnaK/ TS-Zm.DapA-CORgl.dapA.nno/T-Ta.hHsp17> | | | | |
| LH244 | | | | |
| 35. pMON74138: P-Zm.B32/I-Zm.DnaK-Zm.LKR dsRNA-Zm.DnaK/TS-Zm.DapA-CORgl.dapA.nno / TS-Zm.DapA-Ec.lysC/T-Ta.Hsp17> | | | | |
| LH244 | | | | |

**Table 2. Lysine levels measured in R1 events transformed with pMON80378**

| Corn event | Free lysine level (ppm) |
|---|---|
| Zm_M118682 | 16379 |
| Zm_M118685 | 15092 |
| Zm_M118686 | 17878 |
| Zm_M118699 | 18815 |
| Zm_M118700 | 22719 |
| Zm_M118702 | 1832 |

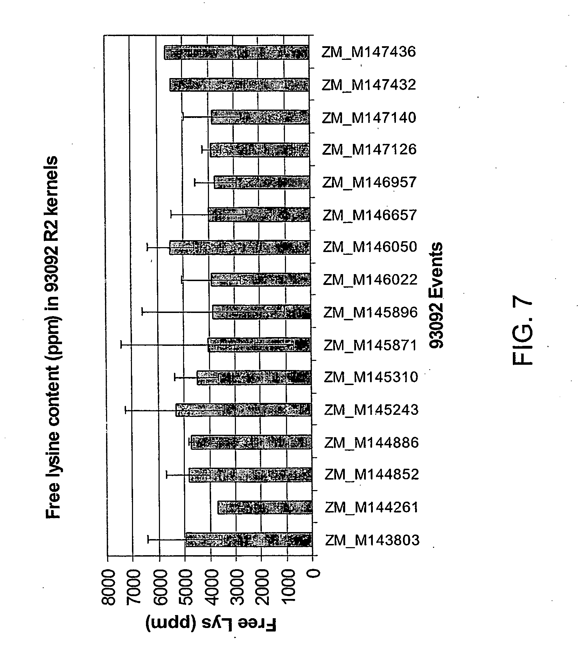

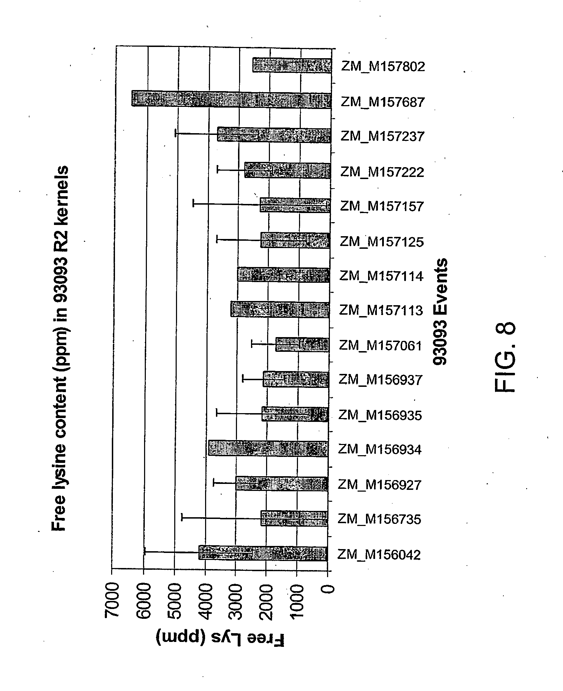

Free lysine content of kernels from events comprising pMON93092 was also determined by tandem mass spectrophotometry (LC MS-MS) on ground and extracted samples of approximately 20 kernels per ear. The amount of free lysine in parts per million (ppm) in R2 kernels from a number of transformation events is shown in FIG. 7 and FIG. 8. For events comprising pMON93092 as shown, levels of free lysine ranged between approximately 3500-5500 ppm, while for events comprising pMON93093 as shown, levels ranged between approximately 1700-6500 ppm. In most cases, two or more ears per event were independently measured and the standard deviation is shown. In comparison, lysine levels for wild type corn average between 50 and 100 ppm.

A deposit by Monsanto Technology LLC, of pMON93092 and pMON93093 disclosed above and recited in the claims, has been made under the Budapest Treaty with the American Type Culture Collection (ATCC), 10801 University Boulevard, Manassas, Va. 20110. The ATCC accession numbers are PTA-6733 and PTA-6734, respectively. The deposit will be maintained in the depository for a period of 30 years, or 5 years after the last request, or for the effective life of the patent, whichever is longer, and will be replaced as necessary during that period.

### REFERENCES

U.S. Patents 4,810,648; 4,957,748; 4,959,317; 5,015,580; 5,034,322; 5,094,945; 5,100,679; 5,107,065; 5,188,642; 5,219,596; 5,258,300; 5,288,300; 5,322,938; 5,352,605; 5,359,142; 5,362,865; 5,367,110; 5,527,695; 5,530,196; 5,538,880; 5,550,318; 5,591,616; 5,627,061; 5,633,435; 5,635,055; 5,641,876; 5,759,829; 5,773,691; 5,780,708; 5,824,877; 5,837,848; 5,936,069; 5,981,840; 6,005,076; 6,040,497; 6,107,549; 6,118,047; 6,140,078; 6,146,669; 6,156,227; 6,160,208; 6,175,060; 6,177,611; 6,232,526; 6,252,138; 6,294,714; 6,376,754; 6,384,301; 6,399,861; 6,403,865; 6,426,446; 6,429,357; 6,429,362; 6,433,252; 6,437,217; 6,459,019; 6,635,806; 6,660,911;
U.S. Patent Appln. Nos. 2002/0112260;. 2003/0056242;. 2003/0106096; 2003/0140364; 20030196219; 2005/0132437; 2005/0193444;
U.S. Patent Serial 09/757,089
Bonnassie et al., Nucleic Acids Res., 18:6421, 1990.
DeBlock et al., EMBO J., 6:2513-2519, 1987.
della-Cioppa et al., Proc. Natl. Acad Sci. USA, 83:6873-6877, 1986.
Fraley et al., Proc. Natl. Acad. Sci. USA, 80:4803-4807, 1983.
Klee et al., Mol. Gen. Genet., 210:437-442, 1987.
Misawa et al., Plant J., 4:833-840, 1993.
Misawa et al., Plant J., 6:481-489, 1994.
Padgette et al., In: Herbicide Resistant Crops, Lewis Publishers, 53-85, 1996. WO 0011200A2
Penaloza-Vazquez et al., Plant Cell Reports, 14:482-487, 1995.
Redenbaugh et al., In: Safety Assessment of Genetically Engineered Flavr Savr™ Tomato, CRC Press, Inc., 1992.
Richaud et al., J. Bacteriol., 166:297-300, 1986.
Sambrook et al., In: Molecular cloning, Cold Spring Harbor Laboratory Press, Cold Spring Harbor, NY, 2000.
Sathasiivan et al., Nucl. Acids Res., 18:2188-2193, 1990.
Turner and Foster, Molec. Biotech., 3:225, 1995.

## Claims

1. A DNA construct comprising a heterologous plant expression cassette encoding a dihydrodipicolinic acid synthase that is substantially resistant to feedback inhibition by free L-lysine and has SEQ ID NO:1, a DNA molecule that is transcribed to produce an RNA molecule that suppresses a lysine ketoglutarate reductase/saccharopine dehydrogenase and is homologous or complementary to SEQ ID NO:2 or comprises a portion thereof sufficient for suppressing the dehydrogenase, and an additional expression cassette comprising a DNA molecule encoding an aspartate kinase that is substantially resistant to feedback inhibition by free L-lysine and has SEQ ID NO:3, wherein each of the DNA molecules is operably linked to a seed enhanced promoter, wherein the promoter promotes the transcription of the DNA molecules substantially in the endosperm of a seed.

2. The construct of claim 1, wherein a DNA molecule encoding a plastid transit signal peptide is operably linked to the DNA molecule encoding the aspartate kinase.

3. The DNA construct of claim 1, wherein DNA molecule encoding a plastid transit signal peptide is operably linked to the DNA molecule encoding dihydrodipicolinic acid synthase.

4. A corn seed comprising native DNA and one or more exogenous plant expression cassettes comprising a DNA molecule encoding a dihydrodipicolinic acid synthase that is substantially resistant to feedback inhibition by free L-lysine and has SEQ ID NO:1, a DNA molecule that is transcribed to produce an RNA molecule that suppresses a lysine ketoglutarate reductase/saccharopine dehydrogenase and is homologous or complementary to SEQ ID NO:2 or comprises a portion thereof sufficient for suppressing the dehydrogenase, and a DNA molecule encoding a lysine feedback resistant aspartate kinase and has SEQ ID NO:3, the DNA molecules being operably linked to one or more promoter molecules that cause transcription of one or more RNA molecules substantially in the endosperm of the corn seed, wherein expression of said cassette results in lysine levels in said corn seed of at least 4,000 ppm.

5. The seed of claim 4, wherein the construct further comprises a DNA molecule that provides for herbicide tolerance, preferably for glyphosate tolerance.

6. The seed of claim 5, wherein the construct encodes a 5-enolpyruvylshikimate 3-phosphate synthase, preferably said 5-enolpyru-vylshikimate 3-phosphate synthase is an aroA:CP4 EPSPS.

7. The seed of claim 4, wherein
(i) the DNA molecule that is transcribed to produce an RNA molecule that suppresses a lysine ketoglutarate reductase/saccharopine dehydrogenase is SEQ ID NO:9; or
(ii) the DNA molecule further comprises a DNA sequence encoding a chloroplast transit signal peptide operably linked to the DNA sequence encoding the dihydrodipicolinic acid synthase or the aspartate kinase; or
(iii) the promoter is selected from *Zea mays* B32, gamma coixin and *Zea mays* L3 promoter.

8. The seed of claim 4, which has a lysine content
(i) higher than corn seed not containing the exogenous plant expression cassettes; or
(ii) of greater than 4,000 ppm.

9. A processed product of the seed of any one of claims 4 to 8, wherein
(i) said product is a feed, flour, meal, or partially purified protein composition; and
(ii) the processed product comprises fragments of the native DNA and fragments of the exogenous plant expression cassettes.

10. A corn cell comprising one or more exogenous plant expression cassettes comprising a DNA molecule encoding a dihydrodipicolinic acid synthase that is substantially resistant to feedback inhibition by free L-lysine and has SEQ ID NO:1, a DNA molecule that is transcribed to produce an RNA molecule that suppresses a lysine ketoglutarate reductase/saccharopine dehydrogenase and is homologous or complementary to SEQ ID NO:2 or comprises a portion thereof sufficient for suppressing the dehydrogenase, and a DNA molecule encoding a lysine feedback resistant aspartate kinase and has SEQ ID NO:3, the DNA molecules being operably linked to one or more promoter molecules that cause transcription of one or more RNA molecules substantially in the endosperm of a corn seed, wherein expression of said cassette results in lysine levels in said corn seed of at least 4,000 ppm.

11. The cell of claim 10, wherein
(i) the construct further comprises a DNA molecule that encodes a 5-enolpyruvylshikimate 3-phosphate synthase, preferably said 5-enolpyruvylshikimate 3-phosphate synthase is an aroA:CP4 EPSPS; or
(ii) the DNA molecule that is transcribed to produce an RNA molecule that suppresses a lysine ketoglutarate reductase/saccharopine dehydrogenase is SEQ ID NO:9 (the bfx sequence); or
(iii) the DNA molecule further comprises a DNA sequence encoding a chloroplast transit signal peptide operably linked to the DNA sequence encoding the dihydrodipicolinic acid synthase or the aspartate kinase; or
(iv) the promoter is a *Zea mays* B32, gamma coixin or *Zea mays* L3 promoter.

12. The cell of claim 10, which has a lysine content
(i) higher than corn seed not containing the exogenous plant expression cassettes; or
(ii) of greater than 4,000 ppm.

13. A processed product containing intact or ruptured cells according to any one of claims 10 to 12, wherein the product is a feed, meal or flour.

14. A method for making grain comprising the steps of:
a) transforming a cell of a plant with the plant expression cassette pMON80378;
b) regenerating said cell into a fertile plant having the expression cassettes in its genome; and
c) harvesting seed from said plant,
whereby the seed has a lysine content higher than seed of the same variety not transformed with said construct.

15. A method of providing nutrition to a human or lower animal comprising feeding the human or lower animal a diet comprising a high lysine corn seed, or a processed product therefrom, comprising
(i) the plant expression cassette pMON80378; or
(ii) fragments of native DNA from the seed and fragments of the plant expression cassette pMON80378.

16. The processed product of claim 9 obtainable by a method comprising the steps of:
a) transforming a cell of a plant with the plant expression cassette pMON80378;
b) regenerating said cell into a fertile plant having the expression cassettes in its genome;
c) harvesting seed from said plant, and
d) processing said seed into an animal feed, or a meal, or a flour;
whereby the seed has a lysine content higher than seed of the same variety not transformed with said construct.

## Patentansprüche

1. DNA-Konstrukt, umfassend eine heterologe Pflanzenexpressionscassette, die eine Dihydrodipicolinsäure-Synthase codiert, welche gegen Produkthemmung durch freies L-Lysin im Wesentlichen resistent ist und die SEQ ID Nr. 1 aufweist, ein DNA-Molekül, das unter Bildung eines RNA-Moleküls transcribiert wird, welches eine Lysinketoglutarat-Reductase/Saccharopin-Dehydrogenase unterdrückt und zu SEQ ID Nr. 2 homolog oder komplementär ist oder einen Teil davon, der ausreicht, um die Dehydrogenase zu unterdrücken, umfasst, und eine zusätzliche Expressionscassette, die ein DNA-Molekül umfasst, das eine Aspartat-Kinase codiert, die gegen Produkthemmung durch freies L-Lysin im Wesentlichen resistent ist und die SEQ ID Nr. 3 aufweist, wobei jedes der DNA-Moleküle funktionell mit einem samenverstärkten Promotor verknüpft ist, wobei der Promotor die Transcription der DNA-Moleküle im Wesentlichen im Endosperm eines Samens fördert.

2. Konstrukt gemäß Anspruch 1, wobei ein DNA-Molekül, das ein Plastidentransitsignalpeptid codiert, funktionell mit dem DNA-Molekül verknüpft ist, das die Aspartat-Kinase codiert.

3. DNA-Konstrukt gemäß Anspruch 1, wobei ein DNA-Molekül, das ein Plastidentransitsignalpeptid codiert, funktionell mit dem DNA-Molekül verknüpft ist, das die Dihydrodipicolinsäure-Synthase codiert.

4. Maissamen, umfassend native DNA und eine oder mehrere exogene Pflanzenexpressionscassetten, die Folgendes umfassen: ein DNA-Molekül, das eine Dihydrodipicolinsäure-Synthase codiert, welche gegen Produkthemmung durch freies L-Lysin im Wesentlichen resistent ist und die SEQ ID Nr. 1 aufweist, ein DNA-Molekül, das unter Bildung eines RNA-Moleküls transcribiert wird, welches eine Lysinketoglutarat-Reductase/Saccharopin-Dehydrogenase unterdrückt und zu SEQ ID Nr. 2 homolog oder komplementär ist oder einen Teil davon, der ausreicht, um die Dehydrogenase zu unterdrücken, umfasst, und ein DNA-Molekül, das eine gegen Produkthemmung durch Lysin resistente Aspartat-Kinase codiert und die SEQ ID Nr. 3 aufweist, wobei die DNA-Moleküle funktionell mit einem oder mehreren Promotormolekülen verknüpft sind, die die Transcription eines oder mehrerer RNA-Moleküle im Wesentlichen im Endosperm des Maissamens verursachen, wobei die Expression der Cassette zu Lysinkonzentrationen in dem Maissamen von wenigstens 4000 ppm führt.

5. Samen gemäß Anspruch 4, wobei das Konstrukt weiterhin ein DNA-Molekül umfasst, das für Herbizidtoleranz, vorzugsweise Glyphosattoleranz, sorgt.

6. Samen gemäß Anspruch 5, wobei das Konstrukt eine 5-Enolpyruvylshikimat-3-phosphat-Synthase codiert, wobei die 5-Enolpyruvylshikimat-3-phosphat-Synthase vorzugsweise ein aroA:CP4-EPSPS ist.

7. Samen gemäß Anspruch 4, wobei
(i) das DNA-Molekül, das unter Bildung eines RNA-Moleküls transcribiert wird, welches eine Lysinketoglutarat-Reductase/Saccharopin-Dehydrogenase unterdrückt, das SEQ ID Nr. 9 aufweist; oder
(ii) das DNA-Molekül weiterhin eine DNA-Sequenz umfasst, die ein Chloroplastentransitsignalpeptid codiert und funktionell mit der DNA-Sequenz verknüpft ist, die die Dihydrodipicolinsäure-Synthase oder die Aspartat-Kinase codiert; oder
(iii) der Promotor aus *Zea-mays-B32,* gamma-Coixin und dem *Zea-mays-*L3-Promotor ausgewählt ist.

8. Samen gemäß Anspruch 4, der einen Lysingehalt aufweist, der
(i) höher ist als bei Maissamen, der die exogenen Pflanzenexpressionscassetten nicht enthält; oder
(ii) mehr als 4000 ppm beträgt.

9. Verarbeitetes Produkt aus dem Samen gemäß einem der Ansprüche 4 bis 8, wobei
(i) das Produkt ein Futter, Mehl, Grieß oder eine partiell gereinigte Proteinzusammensetzung ist; und
(ii) das verarbeitete Produkt Fragmente der nativen DNA und Fragmente der exogenen Pflanzenexpressionscassetten umfasst.

10. Maiszelle, umfassend eine oder mehrere exogene Pflanzenexpressionscassetten, die Folgendes umfassen: ein DNA-Molekül, das eine Dihydrodipicolinsäure-Synthase codiert, welche gegen Produkthemmung durch freies L-Lysin im Wesentlichen resistent ist und die SEQ ID Nr. 1 aufweist, ein DNA-Molekül, das unter Bildung eines RNA-Moleküls transcribiert wird, welches eine Lysinketoglutarat-Reductase/Saccharopin-Dehydrogenase unterdrückt und zu SEQ ID Nr. 2 homolog oder komplementär ist oder einen Teil davon, der ausreicht, um die Dehydrogenase zu unterdrücken, umfasst, und ein DNA-Molekül, das eine gegen Produkthemmung durch Lysin resistente Aspartat-Kinase codiert und die SEQ ID Nr. 3 aufweist, wobei die DNA-Moleküle funktionell mit einem oder mehreren Promotormolekülen verknüpft sind, die die Transcription eines oder mehrerer RNA-Moleküle im Wesentlichen im Endosperm des Maissamens verursachen, wobei die Expression der Cassette zu Lysinkonzentrationen in dem Maissamen von wenigstens 4000 ppm führt.

11. Zelle gemäß Anspruch 10, wobei
(i) das Konstrukt weiterhin ein DNA-Molekül umfasst, das eine 5-Enolpyruvylshikimat-3-phosphat-Synthase codiert, wobei die 5-Enolpyruvylshikimat-3-phosphat-Synthase vorzugsweise ein aroA:CP4-EPSPS ist; oder
(ii) das DNA-Molekül, das unter Bildung eines RNA-Moleküls transcribiert wird, welches eine Lysinketoglutarat-Reductase/Saccharopin-Dehydrogenase unterdrückt, das SEQ ID Nr. 9 (die bfx-Sequenz) aufweist; oder
(iii) das DNA-Molekül weiterhin eine DNA-Sequenz umfasst, die ein Chloroplastentransitsignalpeptid codiert und funktionell mit der DNA-Sequenz verknüpft ist, die die Dihydrodipicolinsäure-Synthase oder die Aspartat-Kinase codiert; oder
(iv) der Promotor ein *Zea-mays-B32-,* gamma-Coixin- oder *Zea-mays-L3-*Promotor ist.

12. Zelle gemäß Anspruch 10, die einen Lysingehalt aufweist, der
(i) höher ist als bei Maissamen, der die exogenen Pflanzenexpressionscassetten nicht enthält; oder
(ii) mehr als 4000 ppm beträgt.

13. Verarbeitetes Produkt, das intakte oder aufgebrochene Zellen gemäß einem der Ansprüche 10 bis 12 enthält, wobei das Produkt ein Futter, Mehl oder Grieß ist.

14. Verfahren zur Herstellung von Getreide, umfassend die Schritte:
a) Transformieren einer Zelle einer Pflanze mit der Pflanzenexpressionscassette pMON80378;
b) Regenerieren der Zelle zu einer fruchtbaren Pflanze, die die Expressionscassetten in ihrem Genom aufweist; und
c) Ernten von Samen von der Pflanze;
wobei der Samen einen Lysingehalt aufweist, der höher ist als bei Samen derselben Sorte, die nicht mit dem Konstrukt transformiert sind.

15. Verfahren zur Bereitstellung einer Ernährung für einen Menschen oder ein niederes Tier, umfassend das Füttern des Menschen oder des niederen Tiers mit einer Nahrung, die einen lysinreichen Maissamen umfasst, oder mit einem daraus verarbeiteten Produkt, umfassend
(i) die Pflanzenexpressionscassette pMON80378; oder
(ii) Fragmente der nativen DNA aus dem Samen und Fragmente der Pflanzenexpressionscassette pMON80378.

16. Verarbeitetes Produkt gemäß Anspruch 9, das nach einem Verfahren erhältlich ist, das die Schritte umfasst:
a) Transformieren einer Zelle einer Pflanze mit der Pflanzenexpressionscassette pMON80378;
b) Regenerieren der Zelle zu einer fruchtbaren Pflanze, die die Expressionscassetten in ihrem Genom aufweist;
c) Ernten von Samen von der Pflanze; und
d) Verarbeiten des Samens zu einem Tierfutter oder einem Mehl oder Grieß;
wobei der Samen einen Lysingehalt aufweist, der höher ist als bei Samen derselben Sorte, die nicht mit dem Konstrukt transformiert sind.

## Revendications

1. Construction d'ADN comprenant une cassette d'expression dans les plantes hétérologue codant pour une acide dihydrodipicolinique synthase qui est sensiblement résistante à une rétro-inhibition par la L-lysine libre et possède SEQ ID NO: 1, une molécule d'ADN qui est transcrite pour produire une molécule d'ARN qui supprime une cétoglutarate de lysine réductase/saccharopine déshydrogénase et est homologue ou complémentaire de SEQ ID NO: 2 ou comprend une partie de celle-ci suffisante pour supprimer la déshydrogénase, et une cassette d'expression supplémentaire comprenant une molécule d'ADN codant pour une aspartate kinase qui est sensiblement résistante à une rétro-inhibition par la L-lysine libre et possède SEQ ID NO: 3, dans laquelle chacune des molécules d'ADN est liée de manière fonctionnelle à un promoteur amélioré de graine, dans lequel le promoteur favorise la transcription des molécules d'ADN essentiellement dans l'endosperme d'une graine.

2. Construction selon la revendication 1, dans laquelle une molécule d'ADN codant pour un peptide signal de transit dans le plaste est liée de manière fonctionnelle à la molécule d'ADN codant pour l'aspartate kinase.

3. Construction d'ADN selon la revendication 1, dans laquelle une molécule d'ADN codant pour un peptide signal de transit dans le plaste est liée de manière fonctionnelle à la molécule d'ADN codant pour une acide dihydrodipicolinique synthase.

4. Graine de maïs comprenant un ADN natif et une ou plusieurs cassettes d'expression dans les plantes exogènes comprenant une molécule d'ADN codant pour une acide dihydrodipicolinique synthase qui est sensiblement résistante à une rétro-inhibition par la L-lysine libre et possède SEQ ID NO: 1, une molécule d'ADN qui est transcrite pour produire une molécule d'ARN qui supprime une cétoglutarate de lysine réductase/saccharopine déshydrogénase et est homologue ou complémentaire de SEQ ID NO: 2 ou comprend une partie de celle-ci suffisante pour supprimer la déshydrogénase, et une molécule d'ADN codant pour une aspartate kinase résistante à une rétro-inhibition par la lysine libre et possède SEQ ID NO: 3, les molécules d'ADN étant liées de manière fonctionnelle à une ou plusieurs molécules promotrices qui provoquent la transcription d'une ou plusieurs molécules d'ARN essentiellement dans l'endosperme de la graine de maïs, dans laquelle l'expression de ladite cassette aboutit à des taux de lysine dans ladite graine de maïs d'au moins 4 000 ppm.

5. Graine selon la revendication 4, dans laquelle la construction comprend en outre une molécule d'ADN qui fournit une tolérance à un herbicide, de préférence une tolérance au glyphosate.

6. Graine selon la revendication 5, dans laquelle la construction code pour une 5-énolpyruvylshikimate 3-phosphate synthase, de préférence ladite 5-énolpyruvylshikimate 3-phosphate synthase est une EPSPS aroA:CP4.

7. Graine selon la revendication 4, dans laquelle
(i) la molécule d'ADN qui est transcrite pour produire une molécule d'ARN qui supprime une cétoglutarate de lysine réductase/saccharopine déshydrogénase est SEQ ID NO: 9 ; ou
(ii) la molécule d'ADN comprend en outre une séquence d'ADN codant pour un peptide signal de transit dans le chloroplaste liée de manière fonctionnelle à la séquence d'ADN codant pour l'acide dihydrodipicolinique synthase ou l'aspartate kinase ; ou
(iii) le promoteur est choisi parmi le promoteur B32 de *Zea mays,* gamma coixine et L3 de *Zea mays.*

8. Graine selon la revendication 4, qui a une teneur en lysine
(i) plus élevée qu'une graine de maïs ne contenant pas les cassettes d'expression dans les plantes exogènes ; ou
(ii) de plus de 4 000 ppm.

9. Produit transformé de la graine selon l'une quelconque des revendications 4 à 8, dans lequel
(i) ledit produit est un aliment, une farine, une semoule, ou une composition de protéine partiellement purifiée ; et
(ii) le produit transformé comprend des fragments de l'ADN natif et des fragments des cassettes d'expression dans les plantes exogènes.

10. Cellule de maïs comprenant une ou plusieurs cassettes d'expression dans les plantes exogènes comprenant une molécule d'ADN codant pour une acide dihydrodipicolinique synthase qui est sensiblement résistante à une rétro-inhibition par la L-lysine libre et possède SEQ ID NO: 1, une molécule d'ADN qui est transcrite pour produire une molécule d'ARN qui supprime une cétoglutarate de lysine réductase/saccharopine déshydrogénase et est homologue ou complémentaire de SEQ ID NO: 2 ou comprend une partie de celle-ci suffisante pour supprimer la déshydrogénase, et une molécule d'ADN codant pour une aspartate kinase résistante à une rétro-inhibition par la L-lysine libre et possède SEQ ID NO: 3, les molécules d'ADN étant liées de manière fonctionnelle à une ou plusieurs molécules promotrices qui provoquent la transcription d'une ou plusieurs molécules d'ARN essentiellement dans l'endosperme d'une graine de maïs, dans laquelle l'expression de ladite cassette aboutit à des taux de lysine dans ladite graine de maïs d'au moins 4 000 ppm.

11. Cellule selon la revendication 10, dans laquelle
(i) la construction comprend en outre une molécule d'ADN qui code pour une 5-énolpyruvylshikimate 3-phosphate synthase, de préférence ladite 5-énolpyruvylshikimate 3-phosphate synthase est une EPSPS aroA:CP4 ; ou
(ii) la molécule d'ADN qui est transcrite pour produire une molécule d'ARN qui supprime une cétoglutarate de lysine réductase/saccharopine déshydrogénase est SEQ ID NO: 9 (la séquence bfx) ; ou
(iii) la molécule d'ADN comprend en outre une séquence d'ADN codant pour un peptide signal de transit dans le chloroplaste liée de manière fonctionnelle à la séquence d'ADN codant pour l'acide dihydrodipicolinique synthase ou l'aspartate kinase ; ou
(iv) le promoteur est un promoteur B32 de *Zea mays,* gamma coixine ou L3 de *Zea mays.*

12. Cellule selon la revendication 10, qui a une teneur en lysine
(i) supérieure à une graine de maïs ne contenant pas les cassettes d'expression dans les plantes exogènes ; ou
(ii) de plus de 4 000 ppm.

13. Produit transformé contenant des cellules intactes ou détruites selon l'une quelconque des revendications 10 à 12, dans lequel le produit est un aliment, une semoule ou une farine.

14. Procédé de fabrication d'un grain comprenant les étapes de :
a) transformation d'une cellule d'une plante avec la cassette d'expression dans les plantes pMON80378 ;
b) régénération de ladite cellule en une plante fertile ayant les cassettes d'expression dans son génome ; et
c) récolte de graines à partir de ladite plante,
par quoi la graine a une teneur en lysine supérieure à une graine de la même variété non transformée avec ladite construction.

15. Procédé de fourniture d'une nutrition à un humain ou un animal inférieur comprenant nourrir l'humain ou l'animal inférieur avec un régime comprenant une graine de maïs à haute teneur en lysine, ou un produit transformé de celle-ci, comprenant
(i) la cassette d'expression dans les plantes pMON80378 ; ou
(ii) des fragments d'ADN natif provenant de la graine et des fragments de la cassette d'expression dans les plantes pMON80378.

16. Produit transformé selon la revendication 9 pouvant être obtenu par un procédé comprenant les étapes de :
a) transformation d'une cellule d'une plante avec la cassette d'expression dans les plantes pMON80378 ;
b) régénération de ladite cellule en une plante fertile ayant les cassettes d'expression dans son génome ;
c) récolte de graine à partir de ladite plante, et
d) traitement de ladite graine en un aliment pour animaux, ou une semoule, ou une farine ;
par quoi la graine a une teneur en lysine supérieure à une graine de la même variété non transformée avec ladite construction.
